# NEW EUROPEAN PATENT SPECIFICATION

(11) **EP 0 827 534 B2**
(45) Date of publication and mention of the opposition decision: **06.05.2009**
(45) Mention of the grant of the patent: 14.07.2004
(21) Application number: 96914993.9
(22) Date of filing: 26.04.1996
(51) Int. Cl.: C12N 9/42, C11D 3/386

(54) **DETERGENTS COMPRISING CELLULASES**
CELLULASEN ENTHALTENDE WASCHMITTEL
DETERGENTS COMPRENANT DES CELLULASES

(30) Priority: 28.04.1995 EP 95201115; 12.03.1996 US 614115
(43) Date of publication of application: 11.03.1998
(60) Divisional application: 02017961.0
(73) Proprietor: Henkel AG & Co. KGaA, 40589 Düsseldorf (DE)
(72) Inventor: LENTING, Hermanus, Bernardus, Maria, NL-2641 VT Pijnacker (NL); VAN BECKHOVEN, Rudolf Franciscus WilhelmusCornelis, NL-4334 EK Breda (NL); MAURER, Karl-Heinz, D-40699 Erkrath (DE); KOTTWITZ, Beatrix, D-40593 Düsseldorf (DE); WEISS, Albrecht, D-40764 Langenfeld (DE); VAN SOLINGEN, Pieter, NL-2671 VZ Naaldwijk (NL)
(86) International application number: PCT/EP1996/001755
(87) International publication number: WO 1996/034092

(56) References cited:
- EP-A- 0 271 004
- EP-A- 0 636 740
- WO-A-94/01532
- WO-A-95/02675
- WO-A-95/18219
- JOURNAL OF BACTERIOLOGY, vol. 168, no. 2, November 1986, pages 479-485, XP000600247 FUMIYASU FUKUMORI ET AL.: "Nucleotide sequences of two cellulase genes from alkalophilic Bacillus sp. strain N-4 and their strong homology" cited in the application
- PREBEN NIELSEN ET AL: 'Comparative 16S rDNA sequence analysis of some alkaliphilic bacilli and the establishment of a sixth rRNA group within the genus Bacillus' FEMS MICROBIOLOGY LETTERS vol. 117, pages 61 - 66
- PREBEN NIELSEN ET AL: 'Phenetic diversity of alkaliphilic Bacillus strains: proposal for nine new species' MICROBIOLOGY vol. 141, pages 1745 - 1761
- SCHLEGEL H.G.: 'Allgemeine Mikrobiologie', 1985, GEORG THIEME VERLAG, STUTTGART
- PEDERSEN G.L. ET AL: 'Biopolishing von Cellulosetextilien' MELLIAND TEXTILBERICHTE vol. 12, 1993, pages 1277 - 1280
- KOKI HORIKOSHI ET AL: 'Alkalophilic Microorganisms. A new microbial world', 1982, JAPAN SCIENTIFIC SOCIETIES PRESS, TOKYO
- STRYER L.: "Biochemistry" page 107, 1988, FREEMAN, NEW YORK

## Description

The present invention relates to the use of novei ceiiuiases with improved properties in detergents and aqueous laundry solutions. The invention further relates to detergents and detergent additives comprising the novel cellulase.

Cellulases, also called cellulolytic enzymes, are enzymes which are capable of the hydrolysis of the β-D-glucosidic linkages in celluloses. Cellulolytic enzymes have been divided traditionally into three classes endoglucanases, exoglucanases or cellobio-hydrolases and β-glucosidases (Knowles, J et al. (1987), TIBTECH 5, 255-261). Cellulolytic enzymes can be produced by a large number of bactena, yeasts and fungi Microorganisms that produce cellulases are descnbed in for example GB-A-2094826.

Several applications have been developed for the use of cellulolytic enzymes:
- degrading (wood)cellulose pulp into sugars for (bio)ethanol production;
- several textile treatments like 'stone washing' and 'biopolishing';
- application in detergent compositions.

The use of cellulases in detergent compositions started with cellulases capable of reducing the harshness, i. e softening, of cotton containing fabrics, as described in for example GB-B-1358599.

It is further known that detergent compositions comprising cellulases are effective in removing dirt, i.e. cleaning. The efficiency of cellulolytic enzymes, cellulases, in terms of cleaning textile has been recognized for some time. GB-A-2075028, GB-A-2095275 and GB-A-2094826 disclose detergent compositions with cellulase for improved cleaning performance.

It is also known in the art that cellulases can act as a colour clarifying agent in laundry detergents. After repeated washing of soiled fabrics, cotton containing fabrics appear to be greyish, most probably due to disrupted fibres caused by mechanical action. The fibres are tom up resulting in disordered fibres which are broken. The use of cellulases as colour clarification agents for coloured fabrics has been described in EP-A-0220016. Actually cellulase mixtures from the fungal strain Humicola insolens (DSM 1800) are commonly used in detergents to result in antipilling and colour revival properties. The cellulolytic enzyme system produced by the wild type microorganism is available under the trade name of Celluzyme® by Novo-Nordisk. In addition a cloned (single) cellulase from the same origin under the trade name Carezyme® is also used in detergents.

The main disadvantage of the cellulases known in the art showing colour clarification is that these enzymes agressively degrade the cellulose containing fabrics which results in damage by undesirable loss of tensile strength of the fabrics.

On the other hand cellulases known in the art showing good cleaning properties show hardly any colour clarification effects. The first commercial detergent with cellulases in the world contained a bacterial cellulase. This enzyme represents an above mentioned alkaline endoglucanase from a Bacillus species that does not attack cellulose fibers. The enzyme is described to give a cleaning effect during washing. No effects with respect to anti-pilling or colour revival have been described for this enzyme.

From the above it will become clear that it is still desirable to provide for improved cellulases in detergent applications. Using mixtures of cellulases, as suggested in international patent application WO-A-95/02675, is supposed to provide the above mentioned performances in laundry washing, but to our knowledge it has not previously been possible to use single enzymes providing all these characteristics when applied in laundry washing.

Surprisingly it has been found that the use of certain single cellulases which are capable of cleaning, antiredeposition, colour clarification and antipilling performance in laundry washing does not at all result in unacceptable damage to the textiles washed.

Accordingly, the present invention relates to the use of a single cellulase, from Bacillus sp. CBS 669.93 or CBS 670.93, as defined in the claims with a ratio of tensile strength loss (TSL, as herein defined) to antipilling properties (AP, as herein defined) below 1 in aqueous laundry solutions.

To measure tensile strength loss is a way to measure damage caused by mechanical stress or enzymatical action on fibers. It is to be understood that for the purpose of the present invention cotton fiber has to be used. The method measures the tensile strength of single fibers under wet conditions. It is described in the German Standard DIN 53, 857, part 1, as well as in the International Standard ISO 2267.

As the effect normally shows up in a significant amount only after about 20 to 25 wash cycles, there is always some tensile strength loss due to the mechanical forces acting on the cotton fiber dunng the washing process. Therefore the tensile strength loss of a control fabric washed without cellulases using the same formulation of detergent and the same type of washing machine and washing programme has to be subtracted. To calibrate the values, a preparation of the (single) endoglucanase V from Humicola insolens (EG V) in equal amounts of enzymatic protein in the detergent is used as a standard and the value of the tensile strength loss for this sample minus the control value of detergent without cellulase is taken as a TSL of 100%. This cellulase EG V has been descnbed for example in the international patent application WO 91/17243. The amount of protein can be measured for example by using the BCA Pierce method as descnbed by R.E. Brown et al. in Anal. Biochem. 1989, vol. 180, p. 136 - 139.

A preparation of an above mentioned Bacillus cellulase available from Kao Corp. under the trade mark KAC® 500 or KAC® 700 may be used as comparison, resulting in general in a very low tensile strength loss as compared to the control washing experiment with no cellulase present.

The attack of cellulases on protruding microfibrils, pills and cotton fluff on the surface of a cotton fabric results in an optically visible removal of that pills. To test the effect, washings are to be performed using a detergent with and without cellulase, as described for the detremination of TSL. The antipilling effect, too, can best be seen after an increasing number of wash cycles. Therefore a number of 15 to 40 wash cycles are generally used to demonstrate this effect of cellulases.

There are three different methods that can be used for quantification of this effect:
1. visual evaluation by a test group (panel)
2. measurement of light reflection (L-value of the CIELAB-system)
3. determination of the cotton fluffs by means of optical measurement

The determination using the L-value of the CIELAB-system [Commission Internationale de l'Éclairage] was described by U. Hotz in Tenside Surf. Det. 1993, vol 30, page 388

The optical measurement system, which is used in the preferred method of determining the antpilling properties, usually consists of a light source, a microscope tube and a CCD colour camera recording the light reflected from the surface of a fabric. Depending on the amount of pills and fluff on the surface of the fabric the amount of reflected light as measured by digital image analysis changes. Such a system can be used to measure quantitatvely the amount of pill and fluff on fabrics, normally after 15 to 40 wash cycles depending on the type and activity of the cellulase added to the detergent. An optical system which can be used to measure the degree of pilling has been described by T. Müller-Kirschbaum and H. Grundmann in SÖFW, vol. 118 (1992), p. 483 - 499.

Whatever method is used to determine the antipilling effect of the cellulase to be tested, the standard cellulase EG V has to be tested under the same conditions and it's effect has to be determined by the same method, taking into account the value resulting from the use of the detergent without cellulase. The value obtained for EG V is taken as AP = 100 %.

As can be seen from this definition, the known cellulase EG V from Humicola insolens has a ratio of TSL to AP of 1. As the above mentioned Bacillus cellulase available from Kao Corp. under the trade mark KAC® 500 or KACO 700 has a low AP and a very low TSL, it can be seen that also the ratio for this cellulase is approximately 1. Cellulases according to the definition given above which may be used according to the invention, especially in detergents, have a ratio of TSL to AP as much as possible below 1, preferably below 0.8 and more particularly in the range of 0.001 to 0.5. A ratio of TSL to AP of for example 0.5 means that only 50 % of tensile strength loss is seen at an enzyme concentration yielding the same antipilling effect as the standard cellulase.

The aqueous laundry solution preferably comprises cellulase according to the definition given above in concentrations of 0.01 mg/l to 0.2 mg/l, more particularly 0.015 mg/l to 0.1 mg/l. These concentrations refer to the weight of cellulolytic protein. In addition all ingredients normally found in laundry solutions can be present.

Another aspect of the present invention is the use of a single cellulase according to the definition given above with a ratio of TSL to AP below 1 to provide an anti-greying effect to fabrics, especially coloured fabrics.

In another aspect of the invention a single cellulase according to the definition given above with a ratio of TSL to AP below 1 is used to provide a softening effect to fabrics.

The present invention also relates to the use of a single cellulase according to the definition given above with a ratio of TSL to AP below 1 to provide colour clarification or to inhibit colour deterioration of fabrics, especially coloured fabrics.

The present invention further relates to the use of a single cellulase according to the definition given above with a ratio of TSL to AP below 1 to inhibit the wrinkling of fabrics and to ease the ironing of fabrics.

We found that the use of a single cellulase according to the definition of the invention, unlike previously known mixtures of cellulases which provide colour clarification, does not degrade cotton to an undesirable level causing tensile strength loss.

It is further found that in using a cellulase of the definition according to the invention, unlike previously known cellulases which provide colour clarification, the enzyme does not accumulate on the fabric after repeated laundry washing.

In another aspect, the invention is directed to detergent compositions, detergent additives and fabric softener compositions comprising a single cellulase according to the definition given above.

As noted before, the present invention generally relates to the use of novel cellulases. However, prior to disclosing this invention in more detail, the following terms will be defined:
"Cellulase" is a genetic name for enzymes acting on cellulose and its derivatives, and hydrolysing them into glucose, cellobiose or cellooligosaccharides.

The term "single" cellulase used herein is intended to mean a cellulase which is produced by one gene.

"Host cell" means a cell which has the capacity to act as a host and expression vehicle for a recombinant DNA vector comprising DNA which encodes for the native protein or a derivative.

The term "cleaning" means the removal of dirt attached to laundry.

The term "pilling" in this respect is the formation of pills and fuzz on the surface of cotton containing fabrics due to broken or disordered fibres.

The term "antipilling" is used to describe the prevention of the formation of pills and fuzz on the surface of cotton containing fabrics as well as the removal of pills and fuzz from cotton containing fabrics. Antipilling normally results in colour clarification when coloured cotton containing fabrics are treated.

The term "colour clarification" in this respect is the reestablishment of the attractive fresh look of coloured fabrics containing or consisting of cellulose based fibres, which have developed a greyish appearance by treatment, especially with laundry detergents, of the coloured fabric.

The term "redeposition" in this respect is deposition of dirt or colour components that were removed from these textiles or fabrics during a laundry washing or textile treatment.

The term "antiredeposition" in this respect is the action of cellulase to prevent or diminish the redeposition of dirt and colour components on the fabric.

By a "laundry solution" is meant an aqueous solution used for washing, rinsing or conditioning, e.g. softening, fabrics.

In a preferred aspect, the present invention relates to the use of a cellulase which is obtainable from microorganisms which are deposited according to the Budapest Treaty on the International Recognition of the Deposits of Microorganisms for the Purposes of Patent Procedures, at the Centraal Bureau voor Schimmelcultures, Baam, The Netherlands on December 23, 1993 under deposition numbers CBS 669.93 and CBS 670.93 (described in international patent application WO-A-95/18219). These strains have been classified as new species of the genus Bacillus, which do not belong to any of the presently known rRNA-groups of Bacillus. As used herein, the deposited species will be referred to as CBS 669.93 and CBS 670.93.

The microorganisms may be obtained for example from water and soil samples collected in alkaline environments such as alkaline soils and soda lakes.

The microorganisms have subsequently been screened using a carboxymethyl cellulose (CMC)-agar diffusion assay. Strains which showed a clearing zone in this test were isolated as potential cellulase producing strains. Genomic gene libraries of the alkali tolerant cellulase producing strains were constructed. Recombinant clones were screened by agar diffusion on CMC-agar. Recombinant clones that showed clearing zones around the colony were isolated. Single cellulases were produced by fermentation of the recombinant clones in 4*YEP-medium for 48 hours at 30°C. The obtained single cellulases, optionally purified as described in Example 1, were tested in the tests defined above to measure TSL and AP.

Surprisingly it was found that the cellulases obtainable from CBS 670.93 or CBS 669.93 show a good performance in both tests and have a ratio of TSL to AP below 1.

In a preferred embodiment of the invention, an approximately 50 kD cellulase (calculated on the basis of the amino acid sequence (SEQ ID No. 2) of the mature protein) derived from CBS 670.93 (referred to as "BCE 103" herein) is used. It has been revealed by analyzing the gene encoding the amino acid sequence of the approximately 50 kD cellulase that this cellulase is 89% identical in sequence and 92.5% similar in sequence to the cellulase CeIA of *Bacillus sp.* N-4 (Fukumori et al., J. Bacter., vol. 168, pp, 479-485) by using the TFastA program (Sequence Analysis Software Package 6.0 of Genetic Computer Group. University of Wisconsin, Biotechnology Center, Madison, Wisconsin) as described by Pearson and Lipman in Proc. Nat. Acad. Sci., vol. 85, pp. 2444-2448 (1988).

In an equally preferred embodiment of the invention, an approximately 63 kD cellulase (calculated on the basis of amino acid sequence of the mature protein) derived from CBS 669.93 (referred to herein as "BCE 113") is used. It has been revealed by analyzing the gene encoding the amino acid sequence of the approximately 63 kD. cellulase that this cellulase is 58 % identical in sequence and 72 % similar in sequence to the cellulase CeIB of Bacillus lautus (Jorgensen et al, Gene, vol. 93 (1990), p. 55-60) by using the TFastA program (Sequence Analysis Software Package 6.0 of Genetic Computer Group, University of Wisconsin, Biotechnology Center, Madison, Wisconsin) as described by Pearson and Lipman in Proc. Nat. Acad. Sci., vol. 85 (1990), p. 2444-2448. The amino acid sequence of BCE 113 is given in SEQ ID No. 3. The present invention further encompasses the use of cellulases, with an amino acid sequence which have greater than 72 %, preferably greater than 80 % and more particularly greater than 90 % sequence identity thereto, and detergents comprising such a cellulase.

A cellulase which may be used in detergents according to the present invention in addition to having a ratio of TSL to AP below 1 usually performs well in the Antiredeposition Test as described in Example 5. Whiteness maintenance of white fabric is measured by a reflectance measurement. The higher the reflectance value, the more effective ' the tested cellulase is in antiredeposition performance. They also perform well in the Softening Test as described in Example 5. Depilling is the removal of fibrils and/or microfibers that are disordered and/or broken which usually make a coloured cotton containing fabric look greyish. The more disordered and/or broken fibrils are removed the better the coloured cotton containing fabrics look. Depilling effectiveness can be judged by panels or can be quantified by an image analysis system, as specified above for the measurement of AP. Cellulases which fulfil the requirement of the ratio defined above usually exhibit the following properties: They show a delta REM of at least 4 units, preferably at least 5 units, in the Anti Redeposition Test as defined in the Examples, and they show a depilling result which is at least comparable to that of the cellulase obtainable from CBS 670.93.

The cellulases which can be used according to the present invention may be produced by a process which can be developed using genetic engineering. As a first step the gene encoding the cellulase of the present invention can be cloned using λ-phage (expression) vectors and E. coli host cells. Alternatively PCR cloning using consensus primers designed on conserved domains may be used. Expression of the gene encoding the cellulase of the present invention in E. coli is shown to give an active protein.

After a first cloning step in E. cold, a cellulase gene can be transferred to a more preferred industrial expression host such as Bacillus or Streptomyces species, a filamentous fungus such as Aspergillus, or a yeast. High level expression and secretion obtainable in these host organisms allows accumulation of the cellulase of the invention in the fermentation medium from which they can subsequently be recovered.

Cellulases according to the invention are preferably used in amounts of 8·10⁻⁵ % by weight (0.8 ppm) to 8·10⁻³ % by weight (80 ppm), more particularly 1·10⁻⁴ % by weight (1 ppm) to 4·10⁻³ % by weight (40 ppm), referring to the cellulolytic protein, in detergents. Detergent compositions comprising a cellulase defined according to the invention may additionally comprise surfactants which may be of the anionic, non-ionic, cationic, amphotenc or zwittenonic type as well as mixtures of these surfactant classes. Detergent compositions of the invention may contain other detergent ingredients known in the art, as e.g. builders, bleaching agents, bleach activators, anti-corrosion agents, sequestenng agents, soil release polymers, perfumes, other enzymes, enzyme stabilizers, etc.

Suitable builders are in particular those from the classes of polycarboxylic acids, more particularly polymeric acrylic acids, methacrylic acids, maleic acids, copolymers thereof and oxidized carbohydrates, as described in international patent application WO-A-93/16110, layer silicates, more particularly bentonites, alumosilicates, more particularly zeolites, crystalline or amorphous alkali metal silicates, more particularly sodium silicate, and alkali metal carbonates, more particularly sodium carbonate. The polycarboxylic acids mentioned are normally used in the form of their alkali metal salts, more particularly in the form of their sodium or potassium salts. The zeolites preferably incorporated are in particular those of the A, P or X type or mixtures thereof. Preferred alkali metal silicates are those with molar ratios of SiO₂ to alkali metal oxide of 1.5 to 3 0. Builders such as these are preferably present in detergents according to the invention in quantities of 20% by weight to 80% by weight.

Nonionic surfactants may be present in the detergents according to the invention, preferably in quantities of not more than 10% by weight and, more preferably, in quantities of 2% by weight to 6% by weight, based on the detergent as a whole. Suitable nonionic surfactants are alkyl polyglycosides containing 10 to 22 carbon atoms in the alkyl component and alkoxylates, particularly ethoxylates and/or propoxylates, of linear or branched C₁O-22 and preferably C₁₂₋₁₈ alcohols. The degree of alkoxylation of the alcohols is between 1 and 20 and preferably between 3 and 10. They may be prepared in known manner by reaction of the corresponding alcohols with the corresponding alkylene oxides. The fatty aicohoi derivatives are particularly suitable, although their branched-chain isomers, more particularly so-called oxoalcohols, may be used for the production of useable alkoxylates. Accordingly, the ethoxylates of primary alcohols containing linear dodecyl, tetradecyl, hexadecyl or octadecyl radicals and mixtures thereof are particularly useful. In addition, corresponding ethoxylation and/or propoxylation products of alkyl amines, vicinal diols and carboxylic acid amides, which correspond to the alcohols mentioned in regard to the alkyl component, and of alkyl phenols containing 5 to 12 carbon atoms in the alkyl component may also be used.

Suitable anionic surfactants are in particular those of the sulfate or sulfonate type, although other types, such as soaps, long-chain N-acyl sarcosinates, salts of fatty acid cyanamides or salts of ether carboxylic acids, which may be obtained from long-chain alkyl or alkyl phenyl polyglycol ethers and chloroacetic acid, may also be used. The anionic surfactants are preferably used in the form of the sodium salts. Surfactants are preferably present in quantities of 2% by weight to 30% by weight and more preferably in quantities of 5% by weight to 20% by weight.

Particularly suitable surfactants of the sulfate type are the sulfuric acid monoesters of long-chain primary alcohols of natural and synthetic origin containing 10 to 20 carbon atoms, i.e the sulfuric acid monoesters of fatty alcohols such as, for example, coconut oil fatty alcohols, tallow fatty alcohols, oleyl alcohol, or the C₁₀₋₂₀ oxoalcohols and those of secondary alcohols of the same chain length. The sulfunc acid monoesters of aliphatic primary alcohols, secondary alcohols and alkyl phenols ethoxylated with 1 to 6 mol ethylene oxide are particularly suitable. Sulfated fatty acid alkanolamides and sulfated fatty acid monoglycendes are also suitable.

The sulfonate-type surfactants are primarily the alkylbenzene sulfonates containing C₉₋₁₅ alkyl groups, sulfosuccinic acid monoesters and diesters containing 6 to 22 carbon atoms in the alcohol components and the esters of a sulfofatty acids, for example the a-sulfonated methyl or ethyl esters of hydrogenated coconut oil, palm kernel oil or tallow fatty acids. Other suitable surfactants of the sulfonate type are the alkane sulfonates obtainable from C₁₂₋₁₈ alkanes by sulfochlorination or sulfoxidation and subsequent hydrolysis or neutralization or by addition of bisulfite onto olefins and also olefin sulfonates, i.e. mixtures of alkene and hydroxyalkane sulfonates and also disulfonates which are obtained, for example, from long-chain monoolefins with a terminal or internal double bond by sulfonation with gaseous sulfur trioxide and subsequent alkaline or acidic hydrolysis of the sulfonavon products.

Bleaching agents are preferably selcted from the type containing peroxygen, as hydrogen peroxide, alkali perborate, alkali percarbonate, alkali persilicate and/or alkali persulfate. Particularly preferred are sodium perborate monohydrate and sodium percarbonate. Bleaching agents may be present in amounts of 5 % by weight to 25 % by weight, more particularly 7 % by weight to 20 % by weight.

Bleach activator compounds include in particular N- or O-acyl compounds, for example polyacylated alkylene diamines, more particularly tetraacetyl ethylene diamine, N-acylated triazines, more particularly 1,5-diacetyl-2,4-dioxohexahydro-1,3,5-triazine, acylated glycolunls, more particularly tetraacetyl glycoluril, N-acylated hydantoins, hydrazides, triazoles, urazoles, diketopiperazines, sulfuryl amides and cyanurates, also carboxylic anhydndes, more particularly phthalic anhydride, carboxylic acid esters, more particularly sodium isononanoyloxy benzene sulfonate, and acylated sugar derivatives, more particularly pentaacetyl glucose. The bleach activator may be coated in the usual way with shell-forming substances or may be granulated, optionally using granulation aids, and if desired may contain other additives, for example dye. A bleach activator which forms peroxocarboxylic acids with 2 to 12 carbon atoms, in particular peroxoacetic acid, under the washing conditions is preferably used. A particularly preferred bleach activator is tetraacetyl ethylene diamine (TAED) granulated with carboxymethyl cellulose with average particle sizes of 0 01 mm to 0 8 mm, which may be produced by the process described in European Patent EP-B-0 037 026. In addition to the above mentioned bleach activators or even subtituting them so-called bleach catalysts may be used, which are transition metal complexes, for example as descnbed in

Enzymes which may be present in the detergents according to the invention, in addition to the cellulase according to the definition, are proteases, lipases, cutinases, amylases, pullulanases, other cellulases, hemicellulases, xylanases, oxidases and/or peroxidases They may be presnt in amounts up to 5 % by weight, preferably 0.2 % by weight to 2 % by weight.

The detergent compositions of the invention may be formulated in any convenient form e.g. as a powder or liquid. For the production of detergents with high apparent density of e.g. 650 g/l to 950 g/l a method using an extrusion step, as described in European patent EP-B-0 486 592, is preferred.

Fabric softening compositions comprising the inventive cellulase may compnse further to this cellulase cationic surfactants, preferably of the so-called esterquat type, which are capable of fabnc softening and which may increase the fabric softening properties of the compositions.

### Examples

### Example 1: Production of cellulases

### - Screening for cellulase producing microorganisms

Two methods were applied for the isolation of cellulase-producing microorganisms:
1) the soil and water samples were suspended in 0.85% saline solution and directly used in the carboxymethyl cellulose (CMC)-agar diffusion assay for detection of cellulase producing colonies.
2) The soil and water samples were enriched for cellulase containing strains by incubation in a cellulose containing liquid minimal medium or GAM-medium for 1 to 3 days at 40°C. Cultures that showed bacterial growth were analyzed for cellulase activity using the CMC-agar diffusion assay for detection of cellulase producing colonies.

### - Isolation of alkalitolerant, cellulase producing strains

Strains that showed clearing zones in the agar diffusion assay were fermented in 25 millilitre GAM-medium in 100 millilitre shake flasks in an Incubator Shaker (New Brunswick Scientific, Edison, NJ, USA), at 250 r.p.m. at 40°C for 72 hours CMCase activity was determined in the culture broth at pH 9 and 40°C.

### - Isolation of cellulase genes

Genomic gene libranes of the alkalitolerant cellulase producing strains were constructed in plasmid pTZ18R (Mead, D.A., et al. (1986) Protein Engineering 1, 67). Recombinant clones were screened by agar diffusion on CM-agar as described by Wood, P.J., et al (1988) Methods in Enzymology 160, 59-74. Strains that showed clearing zones around the colony were isolated. The CMCase activity of the recombinant strains was determined after fermentation for 48 hours at 30°C in 4*YEP-medium. The plasmid DNA of the recombinant strains was isolated and the inserts were characterized by restriction enzyme analysis and nucleotide sequence analysis.

### - Media

The minimal medium (pH 9.7) used in the CMC-agar diffusion assay and the ennchment procedure, consisted of KNO₃ 1%, Yeast extract (Difco) 0.1%, KH₂PO₄ 0.1%, MgSO₄.7H₂O 0.02%, Na₂CO₃ 1%, NaCl 4% and 0.25% CMC (Sigma C-4888). For solidificaton 1 5% agar was added.

The complex medium (GAM) used for enzyme production of the donor strains consisted of Peptone (Difco) 0 5%, Yeast extract (Difco) 0.5%, Glucose.H₂O 1%, KH₂PO₄ 0.1%, MgSO₄.7H₂O 0.02%, Na₂CO₃ 1%, NaCl 4%. The pH was adjusted to 9.5 with 4M HCl after which 1% CMC was added.

The complex medium (4*YEP) used for the enzyme production in E. coli recombinant strains consisted of Yeast extract (Difco) 4%. Peptone (Difco) 8%, lactose 0.2%, 100 µg/ml ampicilline.

### - CMC-agar diffusion assay for colonies

Cell suspensions in 0.85% saline solution were plated on CMC-containing minimal medium. After incubation for 1 to 3 days at 40°C, the plates were replica plated and the parent plate was flooded with 0.1% Congo Red for 15 minutes. The plates were destained with 1M NaCl for 30 minutes. The strains that showed a clearing zone aroung the colony were isolated as potential cellulases producing microorganisms.

### - CMC-agar diffusion assay for liquid fractions

Aliquots of 40 µl of enzyme solution or fermentation broth were pipetted in wells punched out from a layer of 5 mm of minimal medium in a petri dish. After incubation for 16 hours at 40°C cellulase activity was detected by Congo Red / NaCl treatment. The diameter of the clearing zone is a measure for the CMCase activity.

### - Resulting cellulase

These expenments resulted in the isolation of a cellulase producing microorganism which was deposited thereafter as CBS 670.93 The microorganism was classified as a new species of the genus Bacillus. Cloning experiments with the CBS 670 93 strain as a donor strain resulted in the isolation of an E. coli clone which was able to produce a cellulase called BCE 103. The nucleotide sequence of the gene coding for said cellulase was analysed. From the cellulase BCE 103 the N-terminal amino acid sequence was determined using standard methods for obtaining and sequencing peptides (Finlay & Geisow (Eds.), Protein Sequencing - a practical approach, 1989, IRL Press). The amino acid sequence of the cellulase was deduced from the nucleotide sequence, using the N-terminal amino acid sequence for the starting point of the mature protein.

The nucleotide sequence for BCE 103 is shown in SEQ ID No. 1 and the amino acid sequence is shown in SEQ ID No. 2.

### - Purification of the cellulase

After the fermentation the cells were separated from the culture liquid by centrifugation (8000 rpm). The cellulase in the supernatant was precipitated with ammonium sulphate (65% saturation). The precipitate was dissolved in 25 mM phosphate buffer pH 7 + 5 mM EDTA until a conductivity of 7 mS/cm. This solution was applied to a Q-Sepharose FF (diameter 5 cm, length 10 cm) Anion Exchange column, after which the column was washed with 25 mM phosphate buffer pH 7 + 5 mM EDTA until an absorbency of 0.2 AU. A gradient of 0 to 0.5 M NaCl in 25 mM phosphate pH 7 was applied to the column in 80 minutes followed by a gradient from 0.5 to 1 M NaCl in 10 minutes. Depending on which cellulase was applied to the column, elution took place in the first or the second gradient. After elution the column was cleaned (upflow) with 1 M NaOH and equilibrated again with 25 mM phosphate pH 7 + 5 mM EDTA. Depending on the elution the obtained cellulase had a purity of up to about 80%.

### - Characterization

### CMC'ase assay

Assays for cellulase activity were performed using modified methods of the PAHBAH method (Lever M. Anal. Biochem. 1972, 47, 273-279 and Lever M Anal. Biochem 1977,81, 21-27).

### Procedure

A test tube is filled with 250 µl 2 5% CMC in 50 mM glycine buffer pH 9 (CMC-low viscosity is purchased from Sigma) and 250 µl aliquots cellulase, diluted in the appropriate buffer. The test tube is incubated for 30 minutes at 40°C in a waterbath, whereafter 1 5 ml of a daily fresh prepared PAHBAH solution (1% PAHBAH in 100 ml 0.5 M NaOH with 100 µl bismuth solution (containing 48.5 g bismuth nitrate, 28.2 g potassium sodium tartrate and 12.0 g NaOH in 100 ml) is added. The mixture is heated at 70°C for 10 minutes, after which it is cooled on ice for 2 minutes. The absorption is measured at 410 nm. To eliminate the background absorbance of the enzyme samples a control experiment is executed as follows: a tube with substrate is incubated under the same conditions as the test tube. After the incubation 1.5 ml PAHBAH and the enzyme preparation is added (in this order). One unit (U) is defined as the amount of enzyme producing 1 µmol of glucose from CMC equivalent determined as reducing sugars per minute per gram product.

The buffer used for the determination of the pH/temperature profiles is a phosphate/citrate system. The pH/temperature profiles were determined using a fixed enzyme concentration which fits in the linear range of the dose response profile measured at pH 7 and 40°C. This enzyme concentration was used for the measurement of the activities under all other determined conditions.

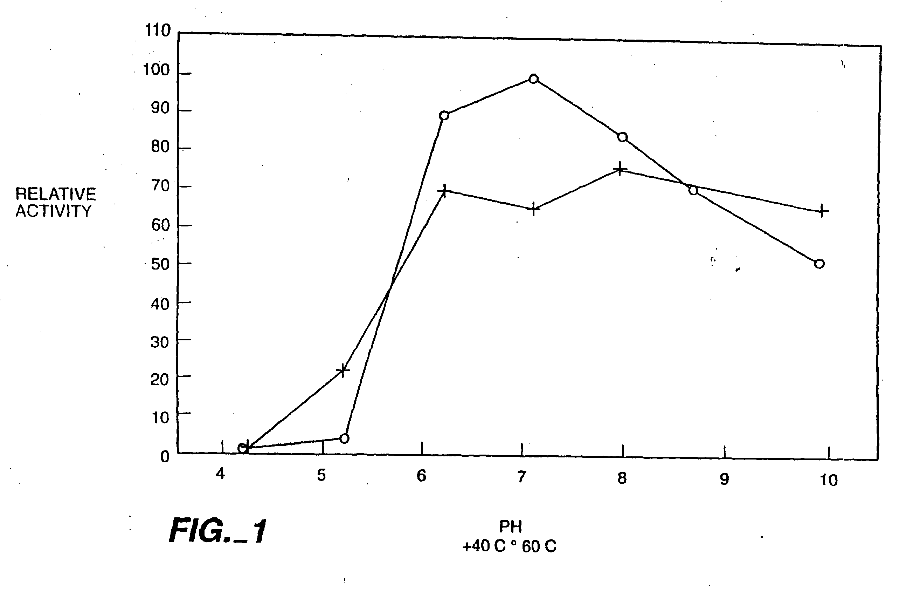

The results for the cellulase BCE 103 are shown in Figure 1. This cellulase shows good activities at alkaline pH, which makes it suitable for application in detergents with an alkaline pH.

### Example 2

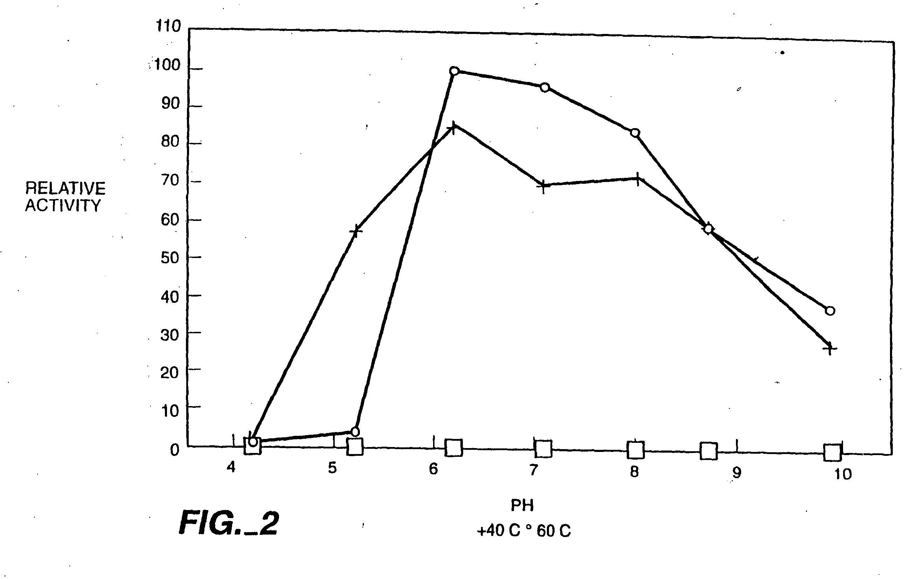

Similar procedures starting with the alkalophilic bacillus strain CBS 669.93 resulted in cellulase BCE 113. The results for this cellulase BCE 113 are shown in Figure 2. This cellulase also shows good activities at alkaline pH, which makes it suitable for application in detergents with an alkaline pH.

### Example 3: Measurement of tensile strength and antipilling

As described for the evaluation of TSL, washing expenments were performed using as detergent matrix a Colour Detergent without bleach, without perfume and enzymes (105 g detergent per wash cycle, pH 10.5), as washing machine a type Miele® W 717, temperature 40°C, program "Normalprogramm", with water of a hardness of 16 °dH (German hardness), wash load 3 5 kg, 25 washes.

Expenments using a composition according to the invention (D1) as well as comparisons (C1 to C3) were run in parallel in identical machines:
- C1:: detergent matrix without cellulase
- C2:: detergent matrix + 0.288 mg endoglucanase V from Humicola insolens
- C3:: detergent matrix + cellulase mixture from Humicola insolens sold as granules Celluzyme® 0.7T
- D1:: detergent matrix + 0.288 mg cellulase BCE 103
- D2:: detergent matrix + 0.288 mg cellulase BCE 113

**Table 1:**

| Results of TSL-measurements [%] | |
|---|---|
| Composition | TSL |
| **C1** | 0 |
| **C2** | 100 |
| **C3** | 38 |
| **D1** | 12 |

Using washing machines of type Miele® W 914, under otherwise identical conditions, gave the following results:

**Table 2:**

| Results of TSL-measurements [%] | |
|---|---|
| Composition | TSL |
| **C1** | 0 |
| **C2** | 100 |
| **D2** | 0.6 |

### Example 4: Measurement of antipilling and Calculation of the ratio TSL to AP

The evaluation of antipilling properties was done with increased concentrations of cellulases for better quantitative evaluation of the effect. A Colour Detergent (5g/l, 10 wash cycles at 40°C) with the addition of cellulase as given in Table 3 was used on "pilled" sweat shirt cotton material (washed 25 times at 90°C with a detergent without cellulase). Evaluation of the pilling was done with the optical measurement system as described before; a degree of pilling of 0 % was assigned to the "pilled" material.

**Table 3:**

| Results of AP-measurements [%] | | | |
|---|---|---|---|
| Enzyme concentration | degree of pilling | | AP [%]of BCE 103 |
| | EG V | BCE 103 | |
| 25 µg/ml | -12.8% | -8.4% | 65% |
| 37.5 µg/ml | -16.0% | -9.6 % | 59% |
| 50 µg/ml | -22.8% | -15.6% | 68% |

An average AP of 64% can be calculated for BCE 103 cellulase. BCE 113 cellulase showed under the same conditions an average AP of 100 %.

Using the values for TSL in Tables 1 and 2, the ratios of TSL to AP for the various cellulases are as in the following Table 4:

**Table 4:**

| Ratio TSL to AP | |
|---|---|
| Enzyme | Ratio |
| EG V | 1 |
| BCE 103 | ≈0.2 |
| BCE 113 | ≈0.02 |

### Example 5: Further test procedures

### - Anti redeposition test

20 ml 0.5% pigmented soil (fresh prepared, daily and consisting of 86% kaolin, 8% soot (Flammruß 101, obtained from Degussa AG), 4% iron oxide black and 2% iron oxide yellow (from Henkel Genthin GmbH)), in a detergent (Persil color® without enzymes, 5 g/l, pH 8.5) was, under agitating (90 rpm) incubated with white cotton fabric (prewashed, 5 cm diameter, obtained from Windelbleiche, Krefeld). Cellulase was added until a final concentration of 1 mU/ml. The mixture was incubated for 30 minutes at 40°C, 90 rpm. As a control the same incubation was carried out without the addition of cellulase. After the incubation the fabric was rinsed thoroughly with running cold water. After drying the whiteness of the fabric was measured by remission (4 measurements per fabric) using a Micro colour Dr. Lange® Colourimeter. The control value was substracted from the sample value. The results, expressed as delta Rem, are shown in Table 5.

### - Fibre Damage Test

One pad of cotton wool (100% cotton, Warenhandels GmbH, Buchholz, Marke Olivia, Selling agency: Aldi) was incubated in 40 ml wash liquor (Persil color® without enzyme, 5 g/l pH 8.5), cellulase at a final concentration of 1 mU/ml was added in a sealed flask and incubated for 20 hours at 40°C under agitation (90 rpm). After the incubation, fibre damage was monitored by the measurement of the quantity of the reducing sugars in solution, using the PAHBAH method described in Example 1. As a control the same incubation was carried out without the addition of cellulase. The results are shown in Table 5.

### - Adsorption Test

White cotton fabnc (Windelbleiche, Bielefeld) prewashed With german Persil® without enzymes at 60°C, was cut round to 9 cm diameter (approx. 0.920 gram). One cotton swatch was incubated in 50 ml 50 mM glycine-NaOH buffer pH 9 including 0.1 % SDS and 1 ml cellulase sample (600 mU/ml) for 60 minutes at 30°C. 2 ml samples were taken at T=0 and at T=60 minutes and were diluted directly (1:2) with 50 mM MES-buffer pH 6 5 and stored at 4°C until measurement. As control the same incubation was carried out without the addition of cotton textile. The activity measurement was determined with a PAHBAH method as descnbed in Example 3, but at pH 6.5 in 50 mM MES buffer. The adsorption was expressed as relative adsorption where the activity apphed at the start of the expenment was set as 100%, T=0 100% activity value - remaining activity (%) = adsorption (%). The results are shown in Table 5

**Table 5:**

| Results of the Antiredeposition Test, Fibre Damage Test and Adsorption Test | | | |
|---|---|---|---|
| Enzyme | Antiredeposition [delta REM] | Fibre Damage [mU] | Adsorption [%] |
| BCE 103 | 5.0 | 0.025 | 7 |
| KAC®²⁾ | 7.5 | 0.006 | 0 |
| EG V | 1.2 | 0.155 | 36 |

### a). Cellulase of Kao Corporation

Cellulase BCE 113 performed in theses tests at least as well as cellulase BCE 103.

### - Softening test

The softness of fabrics treated as in Example 3, but after 15 wash cycles, was rated by an expert panel (5 persons) who awarded grades between 0 (fabric washed 25 times with a detergent without cellulase) and 6 (fabnc prior to any wash) by the feel of the fabrics Compositions as defined in Example 2 were used in the washings. The average rates are given in Table 6 It can be seen that the compositions according to the invention showed the best performance,

**Table 6:**

| Results of the Softening Test | |
|---|---|
| Composition | Rate |
| **C1** | 0 |
| **C2** | 2.1 |
| **C3** | 1.5 |
| **D1** | 2.3 |
| **D2** | 2.2 |

### Legend to the figures

Figure 1 shows the relative activities of the cellulase BCE 103. In Example 1 this figure is referred to as the pH/temperature profiles. All activities for both 40 and 60°C are related to the highest activity which is fixed on 100%.
Figure 2 shows the relative activities of the cellulase BCE 113.

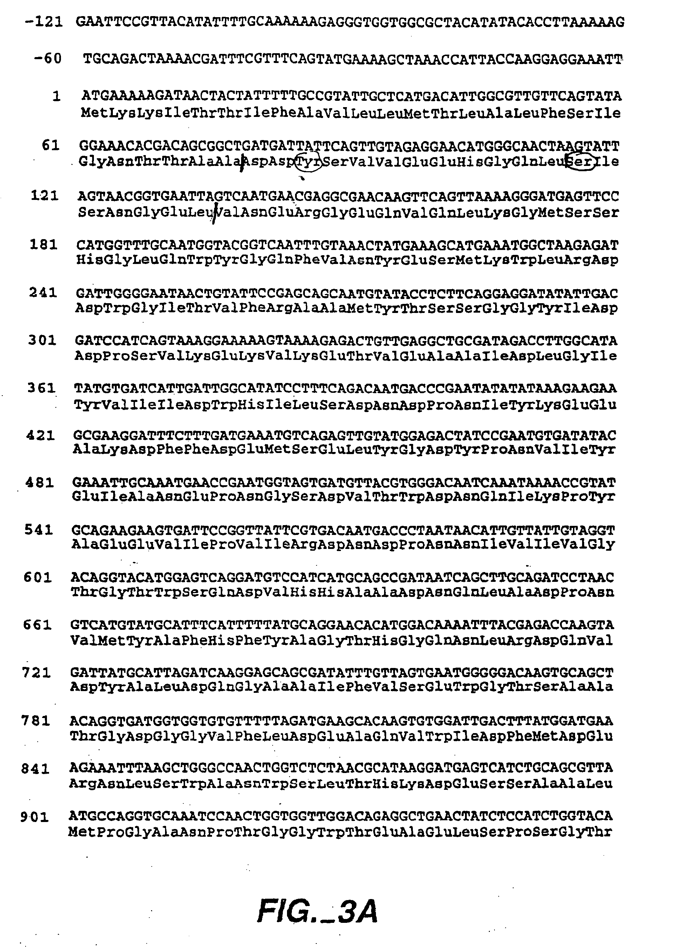

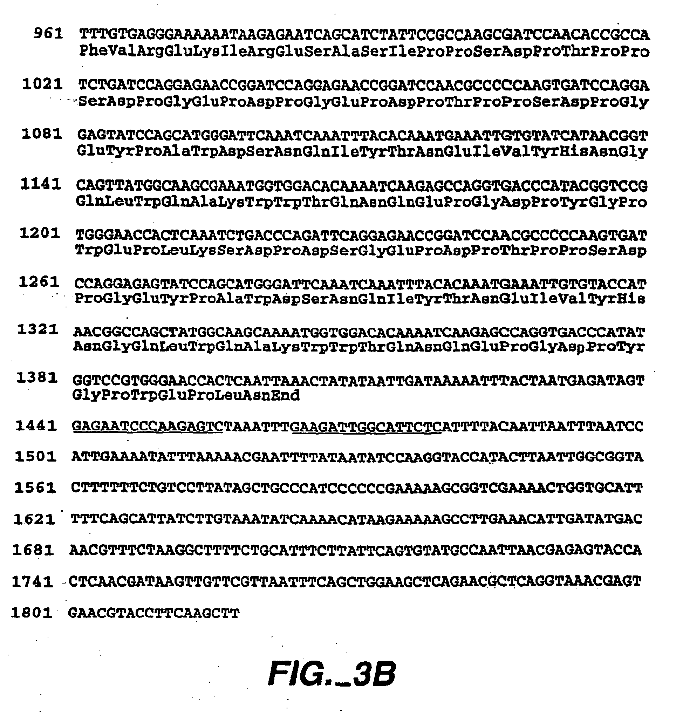

Figure 3 shows the DNA sequence (SEQ ID No. 1) and deduced amino acid sequence (SEQ ID No 2) of the 50 kD cellulase denved from CBS 670.93 with the leader peptide sequence shaded, which upon secretion is cleaved to yield the mature enzyme.

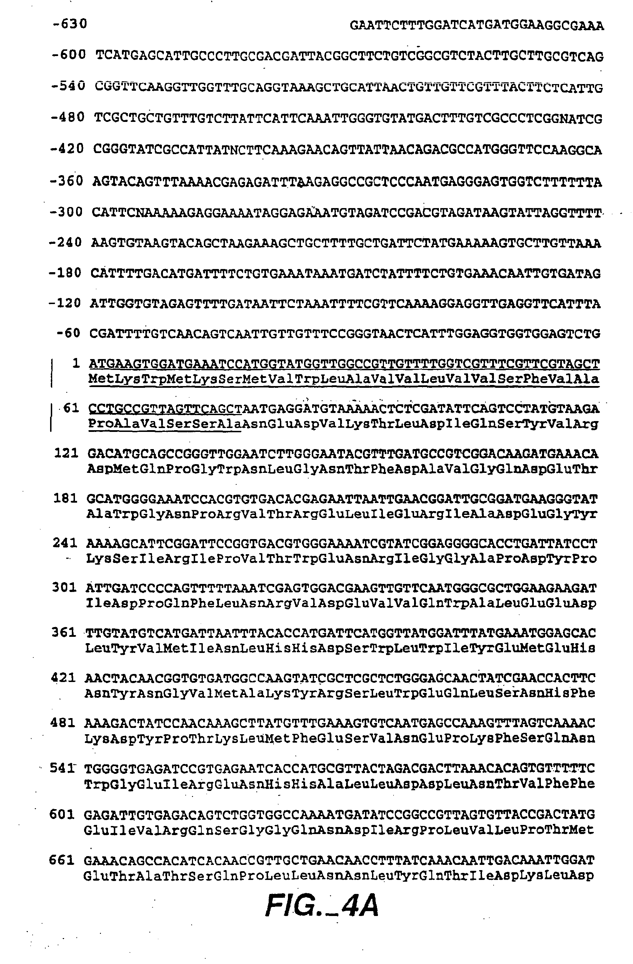

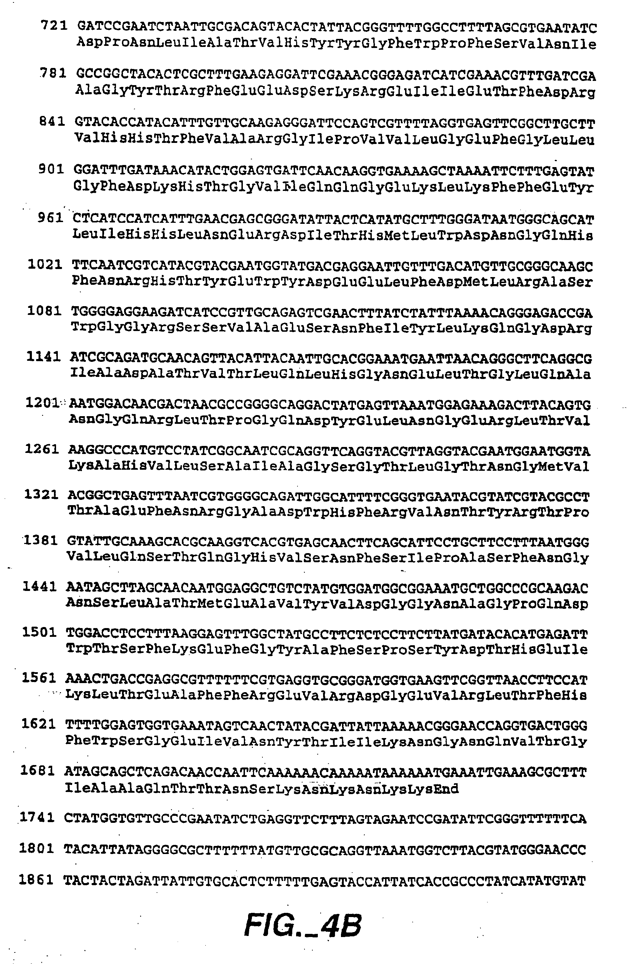

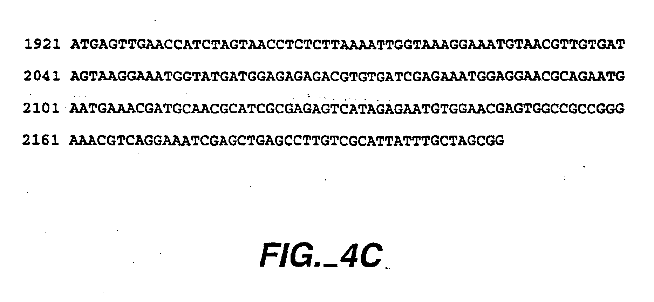

Figure 4 shows the DNA sequence (SEQ ID No 4) and deduced amino acid sequence (SEQ ID No 3) of the 63 kD cellulase derived from CBS 669.93 with the leader peptide sequence underlined, which upon secretion is cleaved to yield the mature enzyme.

## Claims

1. Use of a single cellulase, which has an amino acid sequence with greater than 72% sequence identity to the amino acid sequence as listed in the following (BCE113): 
Met Lys Trp Met Lys Ser Met Val Trp Leu Ala Val Val Leu Val Val
-26 -25 -20 -15

Ser Phe Val Ala Pro Ala Val Ser Ser Ala Asn Glu Asp Val Lys Thr
-10 -5 1 5

Leu Asp Ile Gln Ser Tyr Val Arg Asp Met Gln Pro Gly Trp Asn Leu
10 15 20

Gly Asn Thr Phe Asp Ala Val Gly Gln Asp Glu Thr Ala Trp Gly Asn
25 30 35

Pro Arg Val Thr Arg Glu Leu Ile Glu Arg Ile Ala Asp Glu Gly Tyr
40 45 50

Lys Ser Ile Arg Ile Pro Val Thr Trp Glu Asn Arg Ile Gly Gly Ala
55 60 65 70

Pro Asp Tyr Pro Ile Asp Pro Gln Phe Leu Asn Arg Val Asp Glu Val
75 80 85

Val Gln Trp Ala Leu Glu Glu Asp Leu Tyr Val Met Ile Asn Leu His
90 95 100

His Asp Ser Trp Leu Trp Ile Tyr Glu Met Glu His Asn Tyr Asn Gly
105 110 115

Val Met Ala Lys Tyr Arg Ser Leu Trp Glu Gln Leu Ser Asn His Phe
120 125 130

Lys Asp Tyr Pro Thr Lys Leu Met Phe Glu Ser Val Asn Glu Pro Lys
135 140 145 150

Phe Ser Gln Asn Trp Gly Glu Ile Arg Glu Asn His His Ala Leu Leu
155 160 165

Asp Asp Leu Asn Thr Val Phe Phe Glu Ile Val Arg Gln Ser Gly Gly
170 175 180

Gln Asn Asp Ile Arg Pro Leu Val Leu Pro Thr Met Glu Thr Ala Thr
185 190 195

Ser Gln Pro Leu Leu Asn Asn Leu Tyr Gln Thr Ile Asp Lys Leu Asp
200 205 210

Asp Pro Asn Leu Ile Ala Thr Val His Tyr Tyr Gly Phe Trp Pro Phe
215 220 225 230

Ser Val Asn Ile Ala Gly Tyr Thr Arg Phe Glu Glu Asp Ser Lys Arg
235 240 245
 
Glu Ile Ile Glu Thr Phe Asp Arg Val His His Thr Phe Val Ala Arg
            250                 255                 260

Gly Ile Pro Val Val Leu Gly Glu Phe Gly Leu Leu Gly Phe Asp Lys
        265                 270                 275

His Thr Gly Val Ile Gln Gln Gly Glu Lys Leu Lys Phe Phe Glu Tyr
    280                 285                 290

Leu Ile His His Leu Asn Glu Arg Asp Ile Thr His Met Leu Trp Asp
295                 300                 305                 310

Asn Gly Gln His Phe Asn Arg His Thr Tyr Glu Trp Tyr Asp Glu Glu
                315                 320                 325

Leu Phe Asp Met Leu Arg Ala Ser Trp Gly Gly Arg Ser Ser Val Ala
            330                 335                 340

Glu Ser Asn Phe Ile Tyr Leu Lys Gln Gly Asp Arg Ile Ala Asp Ala
        345                 350                 355

Thr Val Thr Leu Gln Leu His Gly Asn Glu Leu Thr Gly Leu Gln Ala
    360                 365                 370

Asn Gly Gln Arg Leu Thr Pro Gly Gln Asp Tyr Glu Leu Asn Gly Glu
375                 380                 385                 390
 
Arg Leu Thr Val Lys Ala His Val Leu Ser Ala Ile Ala Gly Ser Gly
395 400 405
Thr Leu Gly Thr Asn Gly Met Val Thr Ala Glu Phe Asn Arg Gly Ala
410 415 420
Asp Trp His Phe Arg Val Asn Thr Tyr Arg Thr Pro Val Leu Gln Ser
425 430 435
Thr Gln Gly His Val Ser Asn Phe Ser Ile Pro Ala Ser Phe Asn Gly
440 445 450
Asn Ser Leu Ala Thr Met Glu Ala Val Tyr Val Asp Gly Gly Asn Ala
455 460 465 470
Gly Pro Gln Asp Trp Thr Ser Phe Lys Glu Phe Gly Tyr Ala Phe Ser
475 480 485
Pro Ser Tyr Asp Thr His Glu Ile Lys Leu Thr Glu Ala Phe Phe Arg
490 495 500
Glu Val Arg Asp Gly Glu Val Arg Leu Thr Phe His Phe Trp Ser Gly
505 510 515
Glu Ile Val Asn Tyr Thr Ile Ile Lys Asn Gly Asn Gln Val Thr Gly
520 525 530
Ile Ala Ala Gln Thr Thr Asn Ser Lys Asn Lys Asn Lys Lys *
535 540 545
 or with the amino acid sequence as listed in the following (BCE103): 
Met Lys Lys Ile Thr Thr Ile Phe Ala Val Leu Leu Met Thr Leu Ala
-26 -25 -20 -15

Leu Phe Ser Ile Gly Asn Thr Thr Ala Ala Asp Asp Tyr Ser Val Val
-10 -5 1 5

Glu Glu His Gly Gln Leu Ser Ile Ser Asn Gly Glu Leu Val Asn Glu
10 15 20

Arg Gly Glu Gln Val Gln Leu Lys Gly Met Ser Ser His Gly Leu Gln
25 30 35

Trp Tyr Gly Gln Phe Val Asn Tyr Glu Ser Met Lys Trp Leu Arg Asp
40 45 50

Asp Trp Gly Ile Thr Val Phe Arg Ala Ala Met Tyr Thr Ser Ser Gly
55 60 65 70

Gly Tyr Ile Asp Asp Pro Ser Val Lys Glu Lys Val Lys Glu Thr Val
75 80 85

Glu Ala Ala Ile Asp Leu Gly Ile Tyr Val Ile Ile Asp Trp His Ile
90 95 100

Leu Ser Asp Asn Asp Pro Asn Ile Tyr Lys Glu Glu Ala Lys Asp Phe
105 110 115

Phe Asp Glu Met Ser Glu Leu Tyr Gly Asp Tyr Pro Asn Val Ile Tyr
120 125 130

Glu Ile Ala Asn Glu Pro Asn Gly Ser Asp Val Thr Trp Asp Asn Gln
135 140 145 150

Ile Lys Pro Tyr Ala Glu Glu Val Ile Pro Val Ile Arg Asp Asn Asp
155 160 165

Pro Asn Asn Ile Val Ile Val Gly Thr Gly Thr Trp Ser Gln Asp Val
170 175 180

His His Ala Ala Asp Asn Gln Leu Ala Asp Pro Asn Val Met Tyr Ala
185 190 195

Phe His Phe Tyr Ala Gly Thr His Gly Gln Asn Leu Arg Asp Gln Val
200 205 210

Asp Tyr Ala Leu Asp Gln Gly Ala Ala Ile Phe Val Ser Glu Trp Gly
215 220 225 230

Thr Ser Ala Ala Thr Gly Asp Gly Gly Val Phe Leu Asp Glu Ala Gln
235 240 245

Val Trp Ile Asp Phe Met Asp Glu Arg Asn Leu Ser Trp Ala Asn Trp
250 255 260
 
Ser Leu Thr His Lys Asp Glu Ser Ser Ala Ala Leu Met Pro Gly Ala
265 270 275

Asn Pro Thr Gly Gly Trp Thr Glu Ala Glu Leu Ser Pro Ser Gly Thr
280 285 290

Phe Val Arg Glu Lys Ile Arg Glu Ser Ala Ser Ile Pro Pro Ser Asp
295 300 305 310

Pro Thr Pro Pro Ser Asp Pro Gly Glu Pro Asp Pro Gly Glu Pro Asp
315 320 325

Pro Thr Pro Pro Ser Asp Pro Gly Glu Tyr Pro Ala Trp Asp Ser Asn
330 335 340

Gln Ile Tyr Thr Asn Glu Ile Val Tyr His Asn Gly Gln Leu Trp Gln
345 350 355

Ala Lys Trp Trp Thr Gln Asn Gln Glu Pro Gly Asp Pro Tyr Gly Pro
360 365 370

Trp Glu Pro Leu Lys Ser Asp Pro Asp Ser Gly Glu Pro Asp Pro Thr
375 380 385 390

Pro Pro Ser Asp Pro Gly Glu Tyr Pro Ala Trp Asp Ser Asn Gln Ile
395 400 405

Tyr Thr Asn Glu Ile Val Tyr His Asn Gly Gln Leu Trp Gln Ala Lys
410 415 420

Trp Trp Thr Gln Asn Gln Glu Pro Gly Asp Pro Tyr Gly Pro Trp Glu
425 430 435

Pro Leu Asn *
440
 with a ratio of tensile strength loss (TSL) to antipilling properties (AP) below 1 in aqueous laundry solutions.

2. Use according to claim 1, **characterized in that** the aqueous laundry solution comprises the cellulase in concentrations of 0. 01 mg/l to 0.2 mg/l, more particularly 0.015 mg/l to 0.1 mg/l.

3. Use of a single cellulase according to claim 1 to provide an anti-greying effect to fabrics, especially coloured fabrics.

4. Use of a single cellulase according to claim 1 to provide a softening effect to fabrics.

5. Use of a single cellulase according to claim 1 to provide colour clarification to fabrics, especially coloured fabrics.

6. Use of a single cellulase according to claim 1 to inhibit colour deterioration of fabrics, especially coloured fabrics.

7. Use of a single cellulase according to claim 1 to inhibit the wrinkling of fabrics and to ease the ironing of fabrics.

8. Use according to any of claims 1 to 7, **characterized in that** the ratio of TSL to AP is below 0.8.

9. Use according to any of claims 1 to 8, **characterized in that** the ratio of TSL to AP is in the range of from 0.001 to 0.5.

10. Use according to any of claims 1 to 9, **characterized in that** the cellulase has an amino acid sequence with greater than 90% sequence identity to the amino acid sequence for BCE 113 given in claim
1.

11. Use according to any of claims 1 to 9, **characterized in that** the cellulase has the amino acid sequence as listed in the following:

Met Lys Lys Ile Thr Thr Ile Phe Ala Val Leu Leu Met Thr Leu Ala
-26 -25 -20 -15

Leu Phe Ser Ile Gly Asn Thr Thr Ala Ala Asp Asp Tyr Ser Val Val
-10 -5 1 5

Glu Glu His Gly Gln Leu Ser Ile Ser Asn Gly Glu Leu Val Asn Glu
10 15 20

Arg Gly Glu Gln Val Gln Leu Lys Gly Met Ser Ser His Gly Leu Gln
25 30 35

Trp Tyr Gly Gln Phe Val Asn Tyr Glu Ser Met Lys Trp Leu Arg Asp
40 45 50

Asp Trp Gly Ile Thr Val Phe Arg Ala Ala Met Tyr Thr Ser Ser Gly
55 60 65 70

Gly Tyr Ile Asp Asp Pro Ser Val Lys Glu Lys Val Lys Glu Thr Val
75 80 85

Glu Ala Ala Ile Asp Leu Gly Ile Tyr Val Ile Ile Asp Trp His Ile
90 95 100

Leu Ser Asp Asn Asp Pro Asn Ile Tyr Lys Glu Glu Ala Lys Asp Phe
105 110 115

Phe Asp Glu Met Ser Glu Leu Tyr Gly Asp Tyr Pro Asn Val Ile Tyr
    120                 125                 130

Glu Ile Ala Asn Glu Pro Asn Gly Ser Asp Val Thr Trp Asp Asn Gln
135                 140                 145                 150

Ile Lys Pro Tyr Ala Glu Glu Val Ile Pro Val Ile Arg Asp Asn Asp
                155                 160                 165

Pro Asn Asn Ile Val Ile Val Gly Thr Gly Thr Trp Ser Gln Asp Val
            170                 175                 180

His His Ala Ala Asp Asn Gln Leu Ala Asp Pro Asn Val Met Tyr Ala
        185                 190                 195

Phe His Phe Tyr Ala Gly Thr His Gly Gln Asn Leu Arg Asp Gln Val
    200                 205                 210

Asp Tyr Ala Leu Asp Gln Gly Ala Ala Ile Phe Val Ser Glu Trp Gly
215                 220                 225                 230

Thr Ser Ala Ala Thr Gly Asp Gly Gly Val Phe Leu Asp Glu Ala Gln
                235                 240                 245

Val Trp Ile Asp Phe Met Asp Glu Arg Asn Leu Ser Trp Ala Asn Trp
            250                 255                 260

Ser Leu Thr His Lys Asp Glu Ser Ser Ala Ala Leu Met Pro Gly Ala
        265                 270                 275

Asn Pro Thr Gly Gly Trp Thr Glu Ala Glu Leu Ser Pro Ser Gly Thr
    280                 285                 290

Phe Val Arg Glu Lys Ile Arg Glu Ser Ala Ser Ile Pro Pro Ser Asp
295                 300                 305                 310

Pro Thr Pro Pro Ser Asp Pro Gly Glu Pro Asp Pro Gly Glu Pro Asp
                315                 320                 325

Pro Thr Pro Pro Ser Asp Pro Gly Glu Tyr Pro Ala Trp Asp Ser Asn
            330                 335                 340

Gln Ile Tyr Thr Asn Glu Ile Val Tyr His Asn Gly Gln Leu Trp Gln
        345                 350                 355

Ala Lys Trp Trp Thr Gln Asn Gln Glu Pro Gly Asp Pro Tyr Gly Pro
    360                 365                 370

Trp Glu Pro Leu Lys Ser Asp Pro Asp Ser Gly Glu Pro Asp Pro Thr
375                 380                 385                 390

Pro Pro Ser Asp Pro Gly Glu Tyr Pro Ala Trp Asp Ser Asn Gln Ile
                395                 400                 405

Tyr Thr Asn Glu Ile Val Tyr His Asn Gly Gln Leu Trp Gln Ala Lys
            410                 415                 420

Trp Trp Thr Gln Asn Gln Glu Pro Gly Asp Pro Tyr Gly Pro Trp Glu
        425                 430                 435

Pro Leu Asn  *
    440

12. Use according to any of claims 1 to 9, **characterized in that** the cellulase has the amino acid sequence as listed in the following:

Met Lys Trp Met Lys Ser Met Val Trp Leu Ala Val Val Leu Val Val
-26 -25 -20 -15

Ser Phe Val Ala Pro Ala Val Ser Ser Ala Asn Glu Asp Val Lys Thr
-10 -5 1 5

Leu Asp Ile Gln Ser Tyr Val Arg Asp Met Gln Pro Gly Trp Asn Leu
10 15 20

Gly Asn Thr Phe Asp Ala Val Gly Gln Asp Glu Thr Ala Trp Gly Asn
25 30 35

Pro Arg Val Thr Arg Glu Leu Ile Glu Arg Ile Ala Asp Glu Gly Tyr
40 45 50

Lys Ser Ile Arg Ile Pro Val Thr Trp Glu Asn Arg Ile Gly Gly Ala
55 60 65 70

Pro Asp Tyr Pro Ile Asp Pro Gln Phe Leu Asn Arg Val Asp Glu Val
75 80 85

Val Gln Trp Ala Leu Glu Glu Asp Leu Tyr Val Met Ile Asn Leu His
90 95 100

His Asp Ser Trp Leu Trp Ile Tyr Glu Met Glu His Asn Tyr Asn Gly
105 110 115

Val Met Ala Lys Tyr Arg Ser Leu Trp Glu Gln Leu Ser Asn His Phe
120 125 130

Lys Asp Tyr Pro Thr Lys Leu Met Phe Glu Ser Val Asn Glu Pro Lys
135 140 145 150

Phe Ser Gln Asn Trp Gly Glu Ile Arg Glu Asn His His Ala Leu Leu
155 160 165

Asp Asp Leu Asn Thr Val Phe Phe Glu Ile Val Arg Gln Ser Gly Gly
170 175 180

Gln Asn Asp Ile Arg Pro Leu Val Leu Pro Thr Met Glu Thr Ala Thr
185 190 195

Ser Gln Pro Leu Leu Asn Asn Leu Tyr Gln Thr Ile Asp Lys Leu Asp
200 205 210

Asp Pro Asn Leu Ile Ala Thr Val His Tyr Tyr Gly Phe Trp Pro Phe
215 220 225 230

Ser Val Asn Ile Ala Gly Tyr Thr Arg Phe Glu Glu Asp Ser Lys Arg
235 240 245

Glu Ile Ile Glu Thr Phe Asp Arg Val His His Thr Phe Val Ala Arg
250 255 260

Gly Ile Pro Val Val Leu Gly Glu Phe Gly Leu Leu Gly Phe Asp Lys
265 270 275

His Thr Gly Val Ile Gln Gln Gly Glu Lys Leu Lys Phe Phe Glu Tyr
280 285 290

Leu Ile His His Leu Asn Glu Arg Asp Ile Thr His Met Leu Trp Asp
295                 300                 305                 310

Asn Gly Gln His Phe Asn Arg His Thr Tyr Glu Trp Tyr Asp Glu Glu
                315                 320                 325

Leu Phe Asp Met Leu Arg Ala Ser Trp Gly Gly Arg Ser Ser Val Ala
            330                 335                 340

Glu Ser Asn Phe Ile Tyr Leu Lys Gln Gly Asp Arg Ile Ala Asp Ala
        345                 350                 355

Thr Val Thr Leu Gln Leu His Gly Asn Glu Leu Thr Gly Leu Gln Ala
    360                 365                 370

Asn Gly Gln Arg Leu Thr Pro Gly Gln Asp Tyr Glu Leu Asn Gly Glu
375                 380                 385                 390

Arg Leu Thr Val Lys Ala His Val Leu Ser Ala Ile Ala Gly Ser Gly
                395                 400                 405

Thr Leu Gly Thr Asn Gly Met Val Thr Ala Glu Phe Asn Arg Gly Ala
            410                 415                 420

Asp Trp His Phe Arg Val Asn Thr Tyr Arg Thr Pro Val Leu Gln Ser
        425                 430                 435

Thr Gln Gly His Val Ser Asn Phe Ser Ile Pro Ala Ser Phe Asn Gly
    440                 445                 450

Asn Ser Leu Ala Thr Met Glu Ala Val Tyr Val Asp Gly Gly Asn Ala
455                 460                 465                 470

Gly Pro Gln Asp Trp Thr Ser Phe Lys Glu Phe Gly Tyr Ala Phe Ser
                475                 480                 485

Pro Ser Tyr Asp Thr His Glu Ile Lys Leu Thr Glu Ala Phe Phe Arg
            490                 495                 500

Glu Val Arg Asp Gly Glu Val Arg Leu Thr Phe His Phe Trp Ser Gly
        505                 510                 515

Glu Ile Val Asn Tyr Thr Ile Ile Lys Asn Gly Asn Gln Val Thr Gly
    520                 525                 530

Ile Ala Ala Gln Thr Thr Asn Ser Lys Asn Lys Asn Lys Lys  *
535                 540                 545

13. A detergent composition which comprises a single cellulase, as defined in one of claims 1 or 10 to 12 with a ratio of tensile strength loss (TSL) to antipilling properties (AP) below 1 in aqueous laundry solutions.

14. A detergent composition according to claim 13, **characterized in that** it comprises 0.8 ppm to 80 ppm of the cellulase.

15. A detergents composition according to claim 13, **characterized in that** it comprises 1 ppm to 40 ppm of the cellulase.

## Patentansprüche

1. Verwendung einer einzelnen Cellulase, die eine Aminosäuresequenz mit mehr als 72% Sequenzidentität zu der im Folgenden aufgeführten Aminosäuresequenz (BCE113): 
Met Lys Trp Met Lys Ser Met Val Trp Leu Ala Val Val Leu Val Val
-26 -25 -20 -15

Ser Phe Val Ala Pro Ala Val Ser Ser Ala Asn Glu Asp Val Lys Thr
-10 -5 1 5

Leu Asp Ile Gln Ser Tyr Val Arg Asp Met Gln Pro Gly Trp Asn Leu
10 15 20

Gly Asn Thr Phe Asp Ala Val Gly Gln Asp Glu Thr Ala Trp Gly Asn
25 30 35

Pro Arg Val Thr Arg Glu Leu Ile Glu Arg Ile Ala Asp Glu Gly Tyr
40 45 50

Lys Ser Ile Arg Ile Pro Val Thr Trp Glu Asn Arg Ile Gly Gly Ala
55 60 65 70

Pro Asp Tyr Pro Ile Asp Pro Gln Phe Leu Asn Arg Val Asp Glu Val
75 80 85

Val Gln Trp Ala Leu Glu Glu Asp Leu Tyr Val Met Ile Asn Leu His
90 95 100

His Asp Ser Trp Leu Trp Ile Tyr Glu Met Glu His Asn Tyr Asn Gly
105 110 115

Val Met Ala Lys Tyr Arg Ser Leu Trp Glu Gln Leu Ser Asn His Phe
120 125 130

Lys Asp Tyr Pro Thr Lys Leu Met Phe Glu Ser Val Asn Glu Pro Lys
135 140 145 150

Phe Ser Gln Asn Trp Gly Glu Ile Arg Glu Asn His His Ala Leu Leu
155 160 165

Asp Asp Leu Asn Thr Val Phe Phe Glu Ile Val Arg Gln Ser Gly Gly
170 175 180

Gln Asn Asp Ile Arg Pro Leu Val Leu Pro Thr Met Glu Thr Ala Thr
185 190 195
 
Ser Gln Pro Leu Leu Asn Asn Leu Tyr Gln Thr Ile Asp Lys Leu Asp
200 205 210

Asp Pro Asn Leu Ile Ala Thr Val His Tyr Tyr Gly Phe Trp Pro Phe
215 220 225 230

Ser Val Asn Ile Ala Gly Tyr Thr Arg Phe Glu Glu Asp Ser Lys Arg
235 240 245

Glu Ile Ile Glu Thr Phe Asp Arg Val His His Thr Phe Val Ala Arg
250 255 260

Gly Ile Pro Val Val Leu Gly Glu Phe Gly Leu Leu Gly Phe Asp Lys
265 270 275

His Thr Gly Val Ile Gln Gln Gly Glu Lys Leu Lys Phe Phe Glu Tyr
280 285 290

Leu Ile His His Leu Asn Glu Arg Asp Ile Thr His Met Leu Trp Asp
295 300 305 310

Asn Gly Gln His Phe Asn Arg His Thr Tyr Glu Trp Tyr Asp Glu Glu
315 320 325

Leu Phe Asp Met Leu Arg Ala Ser Trp Gly Gly Arg Ser Ser Val Ala
330 335 340

Glu Ser Asn Phe Ile Tyr Leu Lys Gln Gly Asp Arg Ile Ala Asp Ala
345 350 355

Thr Val Thr Leu Gln Leu His Gly Asn Glu Leu Thr Gly Leu Gln Ala
360 365 370

Asn Gly Gln Arg Leu Thr Pro Gly Gln Asp Tyr Glu Leu Asn Gly Glu
375 380 385 390

Arg Leu Thr Val Lys Ala His Val Leu Ser Ala Ile Ala Gly Ser Gly
395 400 405

Thr Leu Gly Thr Asn Gly Met Val Thr Ala Glu Phe Asn Arg Gly Ala
410 415 420

Asp Trp His Phe Arg Val Asn Thr Tyr Arg Thr Pro Val Leu Gln Ser
425 430 435

Thr Gln Gly His Val Ser Asn Phe Ser Ile Pro Ala Ser Phe Asn Gly
440 445 450

Asn Ser Leu Ala Thr Met Glu Ala Val Tyr Val Asp Gly Gly Asn Ala
455 460 465 470

Gly Pro Gln Asp Trp Thr Ser Phe Lys Glu Phe Gly Tyr Ala Phe Ser
475 480 485
 
Pro Ser Tyr Asp Thr His Glu Ile Lys Leu Thr Glu Ala Phe Phe Arg
490 495 500

Glu Val Arg Asp Gly Glu Val Arg Leu Thr Phe His Phe Trp Ser Gly
505 510 515

Glu Ile Val Asn Tyr Thr Ile Ile Lys Asn Gly Asn Gln Val Thr Gly
520 525 530

Ile Ala Ala Gln Thr Thr Asn Ser Lys Asn Lys Asn Lys Lys *
535 540 545
 und/oder mit der im Folgenden aufgeführten Aminosäuresequenz (BCE103): 
Met Lys Lys Ile Thr Thr Ile Phe Ala Val Leu Leu Met Thr Leu Ala
-26 -25 -20 -15

Leu Phe Ser Ile Gly Asn Thr Thr Ala Ala Asp Asp Tyr Ser Val Val
-10 -5 1 5

Glu Glu His Gly Gln Leu Ser Ile Ser Asn Gly Glu Leu Val Asn Glu
10 15 20

Arg Gly Glu Gln Val Gln Leu Lys Gly Met Ser Ser His Gly Leu Gln
25 30 35

Trp Tyr Gly Gln Phe Val Asn Tyr Glu Ser Met Lys Trp Leu Arg Asp
40 45 50

Asp Trp Gly Ile Thr Val Phe Arg Ala Ala Met Tyr Thr Ser Ser Gly
55 60 65 70

Gly Tyr Ile Asp Asp Pro Ser Val Lys Glu Lys Val Lys Glu Thr Val
75 80 85

Glu Ala Ala Ile Asp Leu Gly Ile Tyr Val Ile Ile Asp Trp His Ile
90 95 100

Leu Ser Asp Asn Asp Pro Asn Ile Tyr Lys Glu Glu Ala Lys Asp Phe
105 110 115

Phe Asp Glu Met Ser Glu Leu Tyr Gly Asp Tyr Pro Asn Val Ile Tyr
120 125 130

Glu Ile Ala Asn Glu Pro Asn Gly Ser Asp Val Thr Trp Asp Asn Gln
135 140 145 150

Ile Lys Pro Tyr Ala Glu Glu Val Ile Pro Val Ile Arg Asp Asn Asp
155 160 165

Pro Asn Asn Ile Val Ile Val Gly Thr Gly Thr Trp Ser Gln Asp Val
170 175 180
 
His His Ala Ala Asp Asn Gln Leu Ala Asp Pro Asn Val Met Tyr Ala
        185                 190                 195

Phe His Phe Tyr Ala Gly Thr His Gly Gln Asn Leu Arg Asp Gln Val
    200                 205                 210

Asp Tyr Ala Leu Asp Gln Gly Ala Ala Ile Phe Val Ser Glu Trp Gly
215                 220                 225                 230

Thr Ser Ala Ala Thr Gly Asp Gly Gly Val Phe Leu Asp Glu Ala Gln
                235                 240                 245

Val Trp Ile Asp Phe Met Asp Glu Arg Asn Leu Ser Trp Ala Asn Trp
            250                 255                 260

Ser Leu Thr His Lys Asp Glu Ser Ser Ala Ala Leu Met Pro Gly Ala
        265                 270                 275

Asn Pro Thr Gly Gly Trp Thr Glu Ala Glu Leu Ser Pro Ser Gly Thr
    280                 285                 290

Phe Val Arg Glu Lys Ile Arg Glu Ser Ala Ser Ile Pro Pro Ser Asp
295                 300                 305                 310

Pro Thr Pro Pro Ser Asp Pro Gly Glu Pro Asp Pro Gly Glu Pro Asp
                315                 320                 325

Pro Thr Pro Pro Ser Asp Pro Gly Glu Tyr Pro Ala Trp Asp Ser Asn
            330                 335                 340

Gln Ile Tyr Thr Asn Glu Ile Val Tyr His Asn Gly Gln Leu Trp Gln
        345                 350                 355

Ala Lys Trp Trp Thr Gln Asn Gln Glu Pro Gly Asp Pro Tyr Gly Pro
    360                 365                 370

Trp Glu Pro Leu Lys Ser Asp Pro Asp Ser Gly Glu Pro Asp Pro Thr
375                 380                 385                 390

Pro Pro Ser Asp Pro Gly Glu Tyr Pro Ala Trp Asp Ser Asn Gln Ile
                395                 400                 405

Tyr Thr Asn Glu Ile Val Tyr His Asn Gly Gln Leu Trp Gln Ala Lys
            410                 415                 420

Trp Trp Thr Gln Asn Gln Glu Pro Gly Asp Pro Tyr Gly Pro Trp Glu
        425                 430                 435

Pro Leu Asn  *
    440
 mit einem Verhältnis zwischen Zugfestigkeitsverlust (Tensile Strength Loss, TSL) und Antipilling-Eigenschaften (AP) von unter 1 in wässrigen Waschlösungen aufweist.

2. Verwendung nach Anspruch 1, **dadurch gekennzeichnet, dass** die wässrige Waschlösung die Cellulase in Konzentrationen von 0,01 mg/l bis 0,2 mg/l, insbesondere 0,015 mg/l bis 0,1 mg/l, umfasst.

3. Verwendung einer einzelnen Cellulase nach Anspruch 1, um eine vergrauungsinhibierende Wirkung auf Textilien, insbesondere farbige Textilien, auszuüben.

4. Verwendung einer einzelnen Cellulase nach Anspruch 1, um eine weich machende Wirkung auf Textilien auszuüben.

5. Verwendung einer einzelnen Cellulase nach Anspruch 1, um eine Farbauffrischung an Textilien, insbesondere farbigen Textilien, zu bewirken.

6. Verwendung einer einzelnen Cellulase nach Anspruch 1, um eine Farbverschlechterung von Textilien, insbesondere farbigen Textilien, zu hemmen.

7. Verwendung einer einzelnen Cellulase nach Anspruch 1, um das Verknittern von Textilien zu hemmen und das Bügeln von Textilien zu erleichtern.

8. Verwendung nach irgendeinem der Ansprüche 1 bis 7, **dadurch gekennzeichnet, dass** das Verhältnis zwischen TSL und AP unter 0,8 liegt.

9. Verwendung nach irgendeinem der Ansprüche 1 bis 8, **dadurch gekennzeichnet, dass** das Verhältnis zwischen TSL und AP im Bereich von 0,001 bis 0,5 liegt.

10. Verwendung nach irgendeinem der Ansprüche 1 bis 9, **dadurch gekennzeichnet, dass** die Cellulase eine Aminosäuresequenz mit mehr als 90% Sequenzidentität zu der in Anspruch 1 angegebenen Aminosäuresequenz für BCE113 aufweist.

11. Verwendung nach irgendeinem der Ansprüche 1 bis 9, **dadurch gekennzeichnet, dass** die Cellulase die im Folgenden aufgeführte Aminosäuresequenz aufweist:

Met Lys Lys Ile Thr Thr Ile Phe Ala Val Leu Leu Met Thr Leu Ala
-26 -25 -20 -15

Leu Phe Ser Ile Gly Asn Thr Thr Ala Ala Asp Asp Tyr Ser Val Val
-10 -5 1 5

Glu Glu His Gly Gln Leu Ser Ile Ser Asn Gly Glu Leu Val Asn Glu
10 15 20

Arg Gly Glu Gln Val Gln Leu Lys Gly Met Ser Ser His Gly Leu Gln
25 30 35

Trp Tyr Gly Gln Phe Val Asn Tyr Glu Ser Met Lys Trp Leu Arg Asp
40 45 50

Asp Trp Gly Ile Thr Val Phe Arg Ala Ala Met Tyr Thr Ser Ser Gly
55 60 65 70

Gly Tyr Ile Asp Asp Pro Ser Val Lys Glu Lys Val Lys Glu Thr Val
75 80 85

Glu Ala Ala Ile Asp Leu Gly Ile Tyr Val Ile Ile Asp Trp His Ile
90 95 100

Leu Ser Asp Asn Asp Pro Asn Ile Tyr Lys Glu Glu Ala Lys Asp Phe
105 110 115

Phe Asp Glu Met Ser Glu Leu Tyr Gly Asp Tyr Pro Asn Val Ile Tyr
    120                 125                 130

Glu Ile Ala Asn Glu Pro Asn Gly Ser Asp Val Thr Trp Asp Asn Gln
135                 140                 145                 150

Ile Lys Pro Tyr Ala Glu Glu Val Ile Pro Val Ile Arg Asp Asn Asp
                155                 160                 165

Pro Asn Asn Ile Val Ile Val Gly Thr Gly Thr Trp Ser Gln Asp Val
            170                 175                 180

His His Ala Ala Asp Asn Gln Leu Ala Asp Pro Asn Val Met Tyr Ala
        185                 190                 195

Phe His Phe Tyr Ala Gly Thr His Gly Gln Asn Leu Arg Asp Gln Val
    200                 205                 210

Asp Tyr Ala Leu Asp Gln Gly Ala Ala Ile Phe Val Ser Glu Trp Gly
215                 220                 225                 230

Thr Ser Ala Ala Thr Gly Asp Gly Gly Val Phe Leu Asp Glu Ala Gln
                235                 240                 245

Val Trp Ile Asp Phe Met Asp Glu Arg Asn Leu Ser Trp Ala Asn Trp
            250                 255                 260

Ser Leu Thr His Lys Asp Glu Ser Ser Ala Ala Leu Met Pro Gly Ala
        265                 270                 275

Asn Pro Thr Gly Gly Trp Thr Glu Ala Glu Leu Ser Pro Ser Gly Thr
    280                 285                 290

Phe Val Arg Glu Lys Ile Arg Glu Ser Ala Ser Ile Pro Pro Ser Asp
295                 300                 305                 310

Pro Thr Pro Pro Ser Asp Pro Gly Glu Pro Asp Pro Gly Glu Pro Asp
                315                 320                 325

Pro Thr Pro Pro Ser Asp Pro Gly Glu Tyr Pro Ala Trp Asp Ser Asn
            330                 335                 340

Gln Ile Tyr Thr Asn Glu Ile Val Tyr His Asn Gly Gln Leu Trp Gln
        345                 350                 355

Ala Lys Trp Trp Thr Gln Asn Gln Glu Pro Gly Asp Pro Tyr Gly Pro
    360                 365                 370

Trp Glu Pro Leu Lys Ser Asp Pro Asp Ser Gly Glu Pro Asp Pro Thr
375                 380                 385                 390

Pro Pro Ser Asp Pro Gly Glu Tyr Pro Ala Trp Asp Ser Asn Gln Ile
                395                 400                 405

Tyr Thr Asn Glu Ile Val Tyr His Asn Gly Gln Leu Trp Gln Ala Lys
            410                 415                 420

Trp Trp Thr Gln Asn Gln Glu Pro Gly Asp Pro Tyr Gly Pro Trp Glu
        425                 430                 435

Pro Leu Asn  *
    440

12. Verwendung nach irgendeinem der Ansprüche 1 bis 9, **dadurch gekennzeichnet, dass** die Cellulase die im Folgenden aufgeführte Aminosäuresequenz aufweist:

Met Lys Trp Met Lys Ser Met Val Trp Leu Ala Val Val Leu Val Val
-26 -25 -20 -15

Ser Phe Val Ala Pro Ala Val Ser Ser Ala Asn Glu Asp Val Lys Thr
-10 -5 1 5

Leu Asp Ile Gln Ser Tyr Val Arg Asp Met Gln Pro Gly Trp Asn Leu
10 15 20

Gly Asn Thr Phe Asp Ala Val Gly Gln Asp Glu Thr Ala Trp Gly Asn
25 30 35

Pro Arg Val Thr Arg Glu Leu Ile Glu Arg Ile Ala Asp Glu Gly Tyr
40 45 50

Lys Ser Ile Arg Ile Pro Val Thr Trp Glu Asn Arg Ile Gly Gly Ala
55 60 65 70

Pro Asp Tyr Pro Ile Asp Pro Gln Phe Leu Asn Arg Val Asp Glu Val
75 80 85

Val Gln Trp Ala Leu Glu Glu Asp Leu Tyr Val Met Ile Asn Leu His
90 95 100

His Asp Ser Trp Leu Trp Ile Tyr Glu Met Glu His Asn Tyr Asn Gly
105 110 115

Val Met Ala Lys Tyr Arg Ser Leu Trp Glu Gln Leu Ser Asn His Phe
120 125 130

Lys Asp Tyr Pro Thr Lys Leu Met Phe Glu Ser Val Asn Glu Pro Lys
135 140 145 150

Phe Ser Gln Asn Trp Gly Glu Ile Arg Glu Asn His His Ala Leu Leu
155 160 165

Asp Asp Leu Asn Thr Val Phe Phe Glu Ile Val Arg Gln Ser Gly Gly
170 175 180

Gln Asn Asp Ile Arg Pro Leu Val Leu Pro Thr Met Glu Thr Ala Thr
185 190 195

Ser Gln Pro Leu Leu Asn Asn Leu Tyr Gln Thr Ile Asp Lys Leu Asp
200 205 210

Asp Pro Asn Leu Ile Ala Thr Val His Tyr Tyr Gly Phe Trp Pro Phe
215 220 225 230

Ser Val Asn Ile Ala Gly Tyr Thr Arg Phe Glu Glu Asp Ser Lys Arg
235 240 245

Glu Ile Ile Glu Thr Phe Asp Arg Val His His Thr Phe Val Ala Arg
250 255 260

Gly Ile Pro Val Val Leu Gly Glu Phe Gly Leu Leu Gly Phe Asp Lys
265 270 275

His Thr Gly Val Ile Gln Gln Gly Glu Lys Leu Lys Phe Phe Glu Tyr
280 285 290

Leu Ile His His Leu Asn Glu Arg Asp Ile Thr His Met Leu Trp Asp
295 300 305 310

Asn Gly Gln His Phe Asn Arg His Thr Tyr Glu Trp Tyr Asp Glu Glu
315 320 325

Leu Phe Asp Met Leu Arg Ala Ser Trp Gly Gly Arg Ser Ser Val Ala
330 335 340

Glu Ser Asn Phe Ile Tyr Leu Lys Gln Gly Asp Arg Ile Ala Asp Ala
345 350 355

Thr Val Thr Leu Gln Leu His Gly Asn Glu Leu Thr Gly Leu Gln Ala
360 365 370

Asn Gly Gln Arg Leu Thr Pro Gly Gln Asp Tyr Glu Leu Asn Gly Glu
375 380 385 390

Arg Leu Thr Val Lys Ala His Val Leu Ser Ala Ile Ala Gly Ser Gly
                395                 400                 405

Thr Leu Gly Thr Asn Gly Met Val Thr Ala Glu Phe Asn Arg Gly Ala
            410                 415                 420

Asp Trp His Phe Arg Val Asn Thr Tyr Arg Thr Pro Val Leu Gln Ser
        425                 430                 435

Thr Gln Gly His Val Ser Asn Phe Ser Ile Pro Ala Ser Phe Asn Gly
    440                 445                 450

Asn Ser Leu Ala Thr Met Glu Ala Val Tyr Val Asp Gly Gly Asn Ala
455                 460                 465                 470

Gly Pro Gln Asp Trp Thr Ser Phe Lys Glu Phe Gly Tyr Ala Phe Ser
                475                 480                 485

Pro Ser Tyr Asp Thr His Glu Ile Lys Leu Thr Glu Ala Phe Phe Arg
            490                 495                 500

Glu Val Arg Asp Gly Glu Val Arg Leu Thr Phe His Phe Trp Ser Gly
        505                 510                 515

Glu Ile Val Asn Tyr Thr Ile Ile Lys Asn Gly Asn Gln Val Thr Gly
    520                 525                 530

Ile Ala Ala Gln Thr Thr Asn Ser Lys Asn Lys Asn Lys Lys  *
535                 540                 545

13. Waschmittelzusammensetzung, die eine einzelne Cellulase nach einem der Ansprüche 1 oder 8 bis 12 mit einem Verhältnis zwischen Zugfestigkeitsverlust (Tensile Strength Loss, TSL) und Antipilling-Eigenschaften (AP) von unter 1 in wässrigen Waschlösungen aufweist.

14. Waschmittelzusammensetzung nach Anspruch 13, **dadurch gekennzeichnet, dass** sie 0,8 ppm bis 80 ppm der Cellulase umfasst.

15. Waschmittelzusammensetzung nach Anspruch 13, **dadurch gekennzeichnet, dass** sie 1 ppm bis 40 ppm der Cellulase umfasst.

## Revendications

1. Utilisation d'une seule cellulase, qui possède une séquence d'acides aminés ayant une identité de séquence supérieure à 72% par rapport à la séquence d'acides aminés telle qu'énumérée dans la suite (BCE113): 
Met Lys Trp Met Lys Ser Met Val Trp Leu Ala Val Val Leu Val Val
-26 -25 -20 -15
Ser Phe Val Ala Pro Ala Val Ser Ser Ala Asn Glu Asp Val Lys Thr
-10 -5 1 5
Leu Asp Ile Gln Ser Tyr Val Arg Asp Met Gln Pro Gly Trp Asn Leu
10 15 20
Gly Asn Thr Phe Asp Ala Val Gly Gln Asp Glu Thr Ala Trp Gly Asn
25 30 35
Pro Arg Val Thr Arg Glu Leu Ile Glu Arg Ile Ala Asp Glu Gly Tyr
40 45 50
Lys Ser Ile Arg Ile Pro Val Thr Trp Glu Asn Arg Ile Gly Gly Ala
55 60 65 70
 
Pro Asp Tyr Pro Ile Asp Pro Gln Phe Leu Asn Arg Val Asp Glu Val
                75                  80                  85

Val Gln Trp Ala Leu Glu Glu Asp Leu Tyr Val Met Ile Asn Leu His
            90                  95                  100

His Asp Ser Trp Leu Trp Ile Tyr Glu Met Glu His Asn Tyr Asn Gly
        105                 110                 115

Val Met Ala Lys Tyr Arg Ser Leu Trp Glu Gln Leu Ser Asn His Phe
    120                 125                 130

Lys Asp Tyr Pro Thr Lys Leu Met Phe Glu Ser Val Asn Glu Pro Lys
135                 140                 145                 150

Phe Ser Gln Asn Trp Gly Glu Ile Arg Glu Asn His His Ala Leu Leu
                155                 160                 165

Asp Asp Leu Asn Thr Val Phe Phe Glu Ile Val Arg Gln Ser Gly Gly
            170                 175                 180

Gln Asn Asp Ile Arg Pro Leu Val Leu Pro Thr Met Glu Thr Ala Thr
        185                 190                 195

Ser Gln Pro Leu Leu Asn Asn Leu Tyr Gln Thr Ile Asp Lys Leu Asp
    200                 205                 210

Asp Pro Asn Leu Ile Ala Thr Val His Tyr Tyr Gly Phe Trp Pro Phe
215                 220                 225                 230

Ser Val Asn Ile Ala Gly Tyr Thr Arg Phe Glu Glu Asp Ser Lys Arg
                235                 240                 245
 
Glu Ile Ile Glu Thr Phe Asp Arg Val His His Thr Phe Val Ala Arg
            250                 255                 260

Gly Ile Pro Val Val Leu Gly Glu Phe Gly Leu Leu Gly Phe Asp Lys
        265                 270                 275

His Thr Gly Val Ile Gln Gln Gly Glu Lys Leu Lys Phe Phe Glu Tyr
    280                 285                 290

Leu Ile His His Leu Asn Glu Arg Asp Ile Thr His Met Leu Trp Asp
295                 300                 305                 310

Asn Gly Gln His Phe Asn Arg His Thr Tyr Glu Trp Tyr Asp Glu Glu
                315                 320                 325

Leu Phe Asp Met Leu Arg Ala Ser Trp Gly Gly Arg Ser Ser Val Ala
            330                 335                 340

Glu Ser Asn Phe Ile Tyr Leu Lys Gln Gly Asp Arg Ile Ala Asp Ala
        345                 350                 355

Thr Val Thr Leu Gln Leu His Gly Asn Glu Leu Thr Gly Leu Gln Ala
    360                 365                 370

Asn Gly Gln Arg Leu Thr Pro Gly Gln Asp Tyr Glu Leu Asn Gly Glu
375                 380                 385                 390
 
Arg Leu Thr Val Lys Ala His Val Leu Ser Ala Ile Ala Gly Ser Gly
                395                 400                 405

Thr Leu Gly Thr Asn Gly Met Val Thr Ala Glu Phe Asn Arg Gly Ala
            410                 415                 420

Asp Trp His Phe Arg Val Asn Thr Tyr Arg Thr Pro Val Leu Gln Ser
        425                 430                 435

Thr Gln Gly His Val Ser Asn Phe Ser Ile Pro Ala Ser Phe Asn Gly
    440                 445                 450

Asn Ser Leu Ala Thr Met Glu Ala Val Tyr Val Asp Gly Gly Asn Ala
455                 460                 465                 470

Gly Pro Gln Asp Trp Thr Ser Phe Lys Glu Phe Gly Tyr Ala Phe Ser
                475                 480                 485

Pro Ser Tyr Asp Thr His Glu Ile Lys Leu Thr Glu Ala Phe Phe Arg
            490                 495                 500

Glu Val Arg Asp Gly Glu Val Arg Leu Thr Phe His Phe Trp Ser Gly
        505                 510                 515

Glu Ile Val Asn Tyr Thr Ile Ile Lys Asn Gly Asn Gln Val Thr Gly
    520                 525                 530

Ile Ala Ala Gln Thr Thr Asn Ser Lys Asn Lys Asn Lys Lys *
535                 540                 545
 et/ou avec la séquence d'acides aminés telle qu'énumérée dans la suite (BCE103): 
Met Lys Lys Ile Thr Thr Ile Phe Ala Val Leu Leu Met Thr Leu Ala
-26 -25 -20 -15

Leu Phe Ser Ile Gly Asn Thr Thr Ala Ala Asp Asp Tyr Ser Val Val
-10 -5 1 5

Glu Glu His Gly Gln Leu Ser Ile Ser Asn Gly Glu Leu Val Asn Glu
10 15 20

Arg Gly Glu Gln Val Gln Leu Lys Gly Met Ser Ser His Gly Leu Gln
25 30 35

Trp Tyr Gly Gln Phe Val Asn Tyr Glu Ser Met Lys Trp Leu Arg Asp
40 45 50

Asp Trp Gly Ile Thr Val Phe Arg Ala Ala Met Tyr Thr Ser Ser Gly
55 60 65 70

Gly Tyr Ile Asp Asp Pro Ser Val Lys Glu Lys Val Lys Glu Thr Val
75 80 85

Glu Ala Ala Ile Asp Leu Gly Ile Tyr Val Ile Ile Asp Trp His Ile
90 95 100

Leu Ser Asp Asn Asp Pro Asn Ile Tyr Lys Glu Glu Ala Lys Asp Phe
105 110 115

Phe Asp Glu Met Ser Glu Leu Tyr Gly Asp Tyr Pro Asn Val Ile Tyr
120 125 130

Glu Ile Ala Asn Glu Pro Asn Gly Ser Asp Val Thr Trp Asp Asn Gln
135 140 145 150

Ile Lys Pro Tyr Ala Glu Glu Val Ile Pro Val Ile Arg Asp Asn Asp
155 160 165

Pro Asn Asn Ile Val Ile Val Gly Thr Gly Thr Trp Ser Gln Asp Val
170 175 180

His His Ala Ala Asp Asn Gln Leu Ala Asp Pro Asn Val Met Tyr Ala
185 190 195

Phe His Phe Tyr Ala Gly Thr His Gly Gln Asn Leu Arg Asp Gln Val
200 205 210

Asp Tyr Ala Leu Asp Gln Gly Ala Ala Ile Phe Val Ser Glu Trp Gly
215 220 225 230

Thr Ser Ala Ala Thr Gly Asp Gly Gly Val Phe Leu Asp Glu Ala Gln
235 240 245

Val Trp Ile Asp Phe Met Asp Glu Arg Asn Leu Ser Trp Ala Asn Trp
250 255 260
 
Ser Leu Thr His Lys Asp Glu Ser Ser Ala Ala Leu Met Pro Gly Ala
        265                 270                 275

Asn Pro Thr Gly Gly Trp Thr Glu Ala Glu Leu Ser Pro Ser Gly Thr
    280                 285                 290

Phe Val Arg Glu Lys Ile Arg Glu Ser Ala Ser Ile Pro Pro Ser Asp
295                 300                 305                 310

Pro Thr Pro Pro Ser Asp Pro Gly Glu Pro Asp Pro Gly Glu Pro Asp
                315                 320                 325

Pro Thr Pro Pro Ser Asp Pro Gly Glu Tyr Pro Ala Trp Asp Ser Asn
            330                 335                 340

Gln Ile Tyr Thr Asn Glu Ile Val Tyr His Asn Gly Gln Leu Trp Gln
        345                 350                 355

Ala Lys Trp Trp Thr Gln Asn Gln Glu Pro Gly Asp Pro Tyr Gly Pro
    360                 365                 370

Trp Glu Pro Leu Lys Ser Asp Pro Asp Ser Gly Glu Pro Asp Pro Thr
375                 380                 385                 390

Pro Pro Ser Asp Pro Gly Glu Tyr Pro Ala Trp Asp Ser Asn Gln Ile
                395                 400                 405

Tyr Thr Asn Glu Ile Val Tyr His Asn Gly Gln Leu Trp Gln Ala Lys
            410                 415                 420

Trp Trp Thr Gln Asn Gln Glu Pro Gly Asp Pro Tyr Gly Pro Trp Glu
        425                 430                 435

Pro Leu Asn *
    440
 ayant un rapport de la perte de résistance à la traction (TSL) aux propriétés anti-boulochage (AP) inférieur à 1 dans des solutions aqueuses de lessive.

2. Utilisation selon la revendication 1, **caractérisée en ce que** la solution aqueuse de lessive comprend la cellulase en des concentrations de 0,01 mg/l à 0,2 mg/l, plus particulièrement de 0,015 mg/l à 0,1 mg/l.

3. Utilisation d'une seule cellulase selon la revendication 1, afin d'exercer un effet anti-grisaille sur les textiles, en particulier les textiles colorés.

4. Utilisation d'une seule cellulase selon la revendication 1, afin d'exercer un effet adoucissant sur les textiles.

5. Utilisation d'une seule cellulase selon la revendication 1, afin de procéder à un ravivement des couleurs des textiles, en particulier des textiles colorés.

6. Utilisation d'une seule cellulase selon la revendication 1, afin d'empêcher la détérioration des couleurs des textiles, en particulier des textiles colorés.

7. Utilisation d'une seule cellulase selon la revendication 1, afin d'empêcher le froissement des textiles et de faciliter le repassage des textiles.

8. Utilisation selon l'une quelconque des revendications 1 à 7, **caractérisée en ce que** le rapport de TSL à AP est inférieur à 0,8.

9. Utilisation selon l'une quelconque des revendications 1 à 8, **caractérisée en ce que** le rapport de TSL à AP est dans la plage de 0,001 à 0,5.

10. Utilisation selon l'une quelconque des revendications 1 à 9, **caractérisée en ce que** la cellulase possède une séquence d'acides aminés ayant une identité de séquence supérieure à 90% par rapport à la séquence d'acides aminés pour BCE113, donnée à la revendication 1.

11. Utilisation selon l'une quelconque des revendications 1 à 9, **caractérisée en ce que** la cellulase possède la séquence d'acides aminés telle qu'énumérée dans la suite:

Met Lys Lys Ile Thr Thr Ile Phe Ala Val Leu Leu Met Thr Leu Ala
-26 -25 -20 -15
Leu Phe Ser Ile Gly Asn Thr Thr Ala Ala Asp Asp Tyr Ser Val Val
-10 -5 1 5
Glu Glu His Gly Gln Leu Ser Ile Ser Asn Gly Glu Leu Val Asn Glu
10 15 20

Arg Gly Glu Gln Val Gln Leu Lys Gly Met Ser Ser His Gly Leu Gln
        25                  30                  35

Trp Tyr Gly Gln Phe Val Asn Tyr Glu Ser Met Lys Trp Leu Arg Asp
    40                  45                  50

Asp Trp Gly Ile Thr Val Phe Arg Ala Ala Met Tyr Thr Ser Ser Gly
55                  60                  65                  70

Gly Tyr Ile Asp Asp Pro Ser Val Lys Glu Lys Val Lys Glu Thr Val
                75                  80                  85

Glu Ala Ala Ile Asp Leu Gly Ile Tyr Val Ile Ile Asp Trp His Ile
            90                  95                  100

Leu Ser Asp Asn Asp Pro Asn Ile Tyr Lys Glu Glu Ala Lys Asp Phe
        105                 110                 115

Phe Asp Glu Met Ser Glu Leu Tyr Gly Asp Tyr Pro Asn Val Ile Tyr
    120                 125                 130

Glu Ile Ala Asn Glu Pro Asn Gly Ser Asp Val Thr Trp Asp Asn Gln
135                 140                 145                 150

Ile Lys Pro Tyr Ala Glu Glu Val Ile Pro Val Ile Arg Asp Asn Asp
                155                 160                 165

Pro Asn Asn Ile Val Ile Val Gly Thr Gly Thr Trp Ser Gln Asp Val
            170                 175                 180

His His Ala Ala Asp Asn Gln Leu Ala Asp Pro Asn Val Met Tyr Ala
        185                 190                 195

Phe His Phe Tyr Ala Gly Thr His Gly Gln Asn Leu Arg Asp Gln Val
    200                 205                 210

Asp Tyr Ala Leu Asp Gln Gly Ala Ala Ile Phe Val Ser Glu Trp Gly
215                 220                 225                 230

Thr Ser Ala Ala Thr Gly Asp Gly Gly Val Phe Leu Asp Glu Ala Gln
                235                 240                 245

Val Trp Ile Asp Phe Met Asp Glu Arg Asn Leu Ser Trp Ala Asn Trp
            250                 255                 260

Ser Leu Thr His Lys Asp Glu Ser Ser Ala Ala Leu Met Pro Gly Ala
        265                 270                 275

Asn Pro Thr Gly Gly Trp Thr Glu Ala Glu Leu Ser Pro Ser Gly Thr
    280                 285                 290

Phe Val Arg Glu Lys Ile Arg Glu Ser Ala Ser Ile Pro Pro Ser Asp
295                 300                 305                 310

Pro Thr Pro Pro Ser Asp Pro Gly Glu Pro Asp Pro Gly Glu Pro Asp
                315                 320                 325

Pro Thr Pro Pro Ser Asp Pro Gly Glu Tyr Pro Ala Trp Asp Ser Asn
            330                 335                 340

Gln Ile Tyr Thr Asn Glu Ile Val Tyr His Asn Gly Gln Leu Trp Gln
345 350 355
Ala Lys Trp Trp Thr Gln Asn Gln Glu Pro Gly Asp Pro Tyr Gly Pro
360 365 370
Trp Glu Pro Leu Lys Ser Asp Pro Asp Ser Gly Glu Pro Asp Pro Thr
375 380 385 390
Pro Pro Ser Asp Pro Gly Glu Tyr Pro Ala Trp Asp Ser Asn Gln Ile
395 400 405
Tyr Thr Asn Glu Ile Val Tyr His Asn Gly Gln Leu Trp Gln Ala Lys
410 415 420
Trp Trp Thr Gln Asn Gln Glu Pro Gly Asp Pro Tyr Gly Pro Trp Glu
425 430 435
Pro Leu Asn *
440

12. Utilisation selon l'une quelconque des revendications 1 à 9, **caractérisée en ce que** la cellulase possède la séquence d'acides aminés telle qu'énumérée dans la suite:

Met Lys Trp Met Lys Ser Met Val Trp Leu Ala Val Val Leu Val Val
-26 -25 -20 -15

Ser Phe Val Ala Pro Ala Val Ser Ser Ala Asn Glu Asp Val Lys Thr
-10 -5 1 5

Leu Asp Ile Gln Ser Tyr Val Arg Asp Met Gln Pro Gly Trp Asn Leu
10 15 20

Gly Asn Thr Phe Asp Ala Val Gly Gln Asp Glu Thr Ala Trp Gly Asn
25 30 35

Pro Arg Val Thr Arg Glu Leu Ile Glu Arg Ile Ala Asp Glu Gly Tyr
40 45 50

Lys Ser Ile Arg Ile Pro Val Thr Trp Glu Asn Arg Ile Gly Gly Ala
55 60 65 70

Pro Asp Tyr Pro Ile Asp Pro Gln Phe Leu Asn Arg Val Asp Glu Val
75 80 85

Val Gln Trp Ala Leu Glu Glu Asp Leu Tyr Val Met Ile Asn Leu His
90 95 100

His Asp Ser Trp Leu Trp Ile Tyr Glu Met Glu His Asn Tyr Asn Gly
105 110 115

Val Met Ala Lys Tyr Arg Ser Leu Trp Glu Gln Leu Ser Asn His Phe
120 125 130

Lys Asp Tyr Pro Thr Lys Leu Met Phe Glu Ser Val Asn Glu Pro Lys
135 140 145 150

Phe Ser Gln Asn Trp Gly Glu Ile Arg Glu Asn His His Ala Leu Leu
155 160 165

Asp Asp Leu Asn Thr Val Phe Phe Glu Ile Val Arg Gln Ser Gly Gly
            170                     175                     180

Gln Asn Asp Ile Arg Pro Leu Val Leu Pro Thr Met Glu Thr Ala Thr
        185                     190                     195

Ser Gln Pro Leu Leu Asn Asn Leu Tyr Gln Thr Ile Asp Lys Leu Asp
    200                     205                     210

Asp Pro Asn Leu Ile Ala Thr Val His Tyr Tyr Gly Phe Trp Pro Phe
215                     220                     225                     230

Ser Val Asn Ile Ala Gly Tyr Thr Arg Phe Glu Glu Asp Ser Lys Arg
                235                     240                     245

Glu Ile Ile Glu Thr Phe Asp Arg Val His His Thr Phe Val Ala Arg
            250                     255                     260

Gly Ile Pro Val Val Leu Gly Glu Phe Gly Leu Leu Gly Phe Asp Lys
        265                     270                     275

His Thr Gly Val Ile Gln Gln Gly Glu Lys Leu Lys Phe Phe Glu Tyr
    280                     285                     290

Leu Ile His His Leu Asn Glu Arg Asp Ile Thr His Met Leu Trp Asp
295                     300                     305                     310

Asn Gly Gln His Phe Asn Arg His Thr Tyr Glu Trp Tyr Asp Glu Glu
                315                     320                     325

Leu Phe Asp Met Leu Arg Ala Ser Trp Gly Gly Arg Ser Ser Val Ala
            330                     335                     340

Glu Ser Asn Phe Ile Tyr Leu Lys Gln Gly Asp Arg Ile Ala Asp Ala
        345                     350                     355

Thr Val Thr Leu Gln Leu His Gly Asn Glu Leu Thr Gly Leu Gln Ala
    360                     365                     370

Asn Gly Gln Arg Leu Thr Pro Gly Gln Asp Tyr Glu Leu Asn Gly Glu
375                     380                     385                     390

Arg Leu Thr Val Lys Ala His Val Leu Ser Ala Ile Ala Gly Ser Gly
                395                     400                     405

Thr Leu Gly Thr Asn Gly Met Val Thr Ala Glu Phe Asn Arg Gly Ala
            410                     415                     420

Asp Trp His Phe Arg Val Asn Thr Tyr Arg Thr Pro Val Leu Gln Ser
        425                     430                     435

Thr Gln Gly His Val Ser Asn Phe Ser Ile Pro Ala Ser Phe Asn Gly
    440                     445                     450

Asn Ser Leu Ala Thr Met Glu Ala Val Tyr Val Asp Gly Gly Asn Ala
455                     460                     465                     470

Gly Pro Gln Asp Trp Thr Ser Phe Lys Glu Phe Gly Tyr Ala Phe Ser
                475                     480                     485

Pro Ser Tyr Asp Thr His Glu Ile Lys Leu Thr Glu Ala Phe Phe Arg
            490                     495                     500

Glu Val Arg Asp Gly Glu Val Arg Leu Thr Phe His Phe Trp Ser Gly
        505                 510                 515

Glu Ile Val Asn Tyr Thr Ile Ile Lys Asn Gly Asn Gln Val Thr Gly
    520                 525                 530

Ile Ala Ala Gln Thr Thr Asn Ser Lys Asn Lys Asn Lys Lys  *
535                 540                 545

13. Composition détergente qui comprend une seule cellulase, telle que définie selon l'une quelconque des revendications 1 ou 8 à 12, ayant un rapport de la perte de résistance à la traction (TSL) aux propriétés anti-boulochage (AP) inférieur à 1 dans les solutions aqueuses de lessive.

14. Composition détergente selon la revendication 13, **caractérisée en ce qu'**elle comprend 0,8 ppm à 80 ppm de la cellulase.

15. Composition détergente selon la revendication 13, **caractérisée en ce qu'**elle comprend 1 ppm à 40 ppm de la cellulase.
